# EUROPEAN PATENT APPLICATION

(11) **EP 4 484 558 A1**
(43) Date of publication of application: **01.01.2025**
(21) Application number: 23814655.9
(22) Date of filing: 17.02.2023
(51) Int. Cl.: C12N 9/22, C12N 15/113, C12N 15/90, C07K 14/00

(54) **OPTIMIZED CAS PROTEIN AND USE THEREOF**

(30) Priority: 31.05.2022 CN 202210603607; 25.10.2022 CN 202211326596
(71) Applicant: Shandong Shunfeng Biotechnology Co., Ltd., Jinan, Shandong 250000 (CN)
(72) Inventor: DUAN, Zhiqiang, Jinan, Shandong 250000 (CN); LIANG, Yafeng, Jinan, Shandong 250000 (CN)
(74) Representative: Bayramoglu et al.
(86) International application number: PCT/CN2023/076767
(87) International publication number: WO 2023/231456

(57) **Abstract**

The present invention belongs to the field of nucleic acid editing, and particularly relates to the technical field of clustered regularly interspaced short palindromic repeats (CRISPR). Specifically, provided are an optimized Cas protein and a use thereof, and the optimized Cas protein has broad application prospects.

## Description

### CROSS REFERENCE TO THE RELATED APPLICATIONS

This application claims priority to Chinese Patent Application No. 202210603607.3 entitled "An Optimized Cas Protein and Use Thereof" filed on May 31, 2022, and Chinese Patent Application No. 202211326596.5 entitled "Cas Protein with Improved Editing Activity and Use Thereof" filed on October 25, 2022, the entire contents of which are incorporated herein by reference.

### TECHNICAL FIELD

The invention relates to the field of gene editing, in particular to the field of clustered regularly interspaced short palindromic repeats (CRISPR) technology. Specifically, the invention relates to an optimized CRISPR-associated protein (Cas) protein and use thereof, in particular to a Cas protein with improved activity and use thereof.

### BACKGROUND TECHNOLOGY

CRISPR/Cas technology is a widely used gene editing technology that specifically binds target sequences on the genome by RNA guidance and cuts DNA to produce a double strand break. It uses biological non-homologous end joining or homologous recombination for site-directed gene editing.

The CRISPR/Cas9 system, the most commonly used type II CRISPR system, recognizes the protospacer adjacent motif (PAM) of 3'-NGG and performs blunt end cutting on the target sequence. The type V CRISPR/Cas system is a newly discovered class of CRISPR systems with a 5'-TTN motif for sticky end cutting of the target sequence, such as Cpf1, C2c1, CasX, and CasY. However, the different CRISPR/Cas currently in existence have different advantages and disadvantages. For example, Cas9, C2c1, and CasX all require two RNAs as guide RNA, while Cpf1 requires only one guide RNA and can be used for multiple gene editing. CasX has a size of 980 amino acids, while the common Cas9, C2c1, CasY, and Cpf1 are usually around 1300 amino acids in size. In addition, the PAM sequences of Cas9, Cpf1, CasX, and CasY are all complex and diverse, while C2c1 recognizes the rigorous 5'-TTN, so its target site is easier to predict than those of other systems, thereby reducing the potential off-target effect.

Chinese invention patent CN111757889B discloses a Cas protein, Cas12f.4, and further discloses that the protein can perform gene editing in eukaryotic cells, but its editing activity is not high. In order to improve the editing efficiency of the protein, this application has optimized the protein and improved its editing efficiency in eukaryotic cells.

### CONTENT OF THE INVENTION

After a lot of experiments and repeated explorations, the inventor of this application has improved its editing activity and expanded its application range through site-directed mutagenesis of Cas12f.4 (referred to as Cas12i3 or Cas12i.3 in this application) protein.

### Cas effector protein

On the one hand, the invention provides an optimized Cas mutant protein; compared to an amino acid sequence of a parent Cas protein, the Cas mutant protein has mutations at any one or more of the following amino acid sites corresponding to amino acid sequence shown in SEQ ID No. 1: 7th and 124th sites.

In one embodiment, the Cas mutant protein is mutated at the 7th amino acid site; further, on the basis of the 7th amino acid mutation, it also includes the 124th amino acid site mutation.

In one embodiment, the Cas mutant protein is mutated at the 124th amino acid site; further, on the basis of the 124th amino acid mutation, it also includes the 7th amino acid site mutation.

In one embodiment, the 7th amino acid is mutated to an amino acid other than S, for example, A, V, G, L, Q, F, W, Y, D, K, E, N, M, T, C, P, H, R, I; preferably, R, H, K, M, F, P, A, W, I, V, L, Q, C, or Y

In one embodiment, the 124th amino acid is mutated to an amino acid other than Y, for example, A, V, G, L, Q, F, W, S, D, K, E, N, M, T, C, P, H, R, I; preferably, R, H, K, M, F, P, A, W, I, V, L, Q, or C.

In some embodiments, the parent Cas protein is a natural wild-type Cas protein; in other embodiments, the parent Cas protein is an engineered Cas protein.

Cas proteins or Cas12i proteins from a variety of organisms can be used as the parent Cas protein, and in some embodiments, the parent Cas protein or the Cas12i protein has nuclease activity. In some embodiments, the parent Cas protein is a nuclease that cuts two strands of a target double-helical nucleic acid (e.g., double-helical DNA). In some embodiments, the parent Cas protein is a nickase that cuts a single strand of the target double-helical nucleic acid (e.g., double-helical DNA).

In one embodiment, the parent Cas protein is a Cas protein of the Cas12 family, preferably a Cas protein of the Cas12i family, for example, Cas12i1, Cas12i2, Cas12i3, etc.

In one embodiment, the amino acid sequence of the Cas protein of the Cas12 family, compared to SEQ ID No. 1, has a sequence identity of at least 70%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% or 100%.

In one embodiment, the amino acid sequence of the parent Cas protein, compared to SEQ ID No. 1, has a sequence identity of at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9%.

In one embodiment, the Cas mutant protein is selected from any one of the following I-III groups:

I, a Cas mutant protein by mutating the amino acid sequence shown in SEQ ID No. 1 at any one or more of the following amino acid sites: 7th and 124th sites;

II, compared to the Cas mutant protein described in I, a Cas mutant protein has the mutation site described in I and has a sequence identity of at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% compared to the Cas mutant protein described in I; and

III, compared to the Cas mutant protein described in I, a sequence has the mutation site described in I and has a substitution, a deletion, or an addition of one or more amino acids compared with the Cas mutant protein described in I; the one or more amino acids include the substitution, the deletion, or the addition of one, two, three, four, five, six, seven, eight, nine, or ten amino acids.

On the other hand, the invention also provides a Cas mutant protein with improved editing activity. Compared to an amino acid sequence of a parent Cas protein, the Cas mutant protein has mutation at the 7th amino acid site corresponding to amino acid sequence shown in SEQ ID No. 1, furthermore, the mutant protein also has a mutation at any one or more of the following amino acid sites corresponding to amino acid sequence shown in SEQ ID No. 1: 233rd, 267th, 369th, 433rd, 168th, 328th, 505th site; preferably, any of two, three, four, five, six, or seven.

In the preferred embodiment, compared to an amino acid sequence of a parent Cas protein, the Cas mutant protein with improved editing activity has mutation at the 7th amino acid site corresponding to amino acid sequence shown in SEQ ID No. 1, furthermore, the mutant protein also has a mutation at the following amino acid sites corresponding to amino acid sequence shown in SEQ ID No. 1:
168th amino acid;
or, 233rd amino acid;
or, 168th amino acid and 267th amino acid are mutated simultaneously;
or, 168th amino acid and 505th amino acid are mutated simultaneously;
or, 233rd amino acid and 267th amino acid are mutated simultaneously;
or, 233rd amino acid and 505th amino acid are mutated simultaneously;
or, 233rd amino acid, 369th amino acid, and 433rd amino acid are mutated simultaneously;
or, 233rd amino acid, 267th amino acid, 328th amino acid, and 369th amino acid are mutated simultaneously;
or, 233rd amino acid, 267th amino acid, 369th amino acid, and 433rd amino acid are mutated simultaneously;
or, 168th amino acid, 267th amino acid, 328th amino acid, and 369th amino acid are mutated simultaneously.

Preferably, the 7th amino acid is mutated to an amino acid other than S, for example, A, V, G, L, Q, F, W, Y, D, K, E, N, M, T, C, P, H, R, I; preferably, R, H, K, M, F, P, A, W, I, V, L, Q, C, or Y; more preferably, R.

In one embodiment, the 168th amino acid is mutated to an amino acid other than N, for example, A, V, G, L, Q, F, W, Y, D, S, E, K, M, T, C, P, H, R, I; preferably, it is mutated to R.

In one embodiment, the 233rd amino acid or the 267th amino acid is mutated to an amino acid other than D, for example, A, V, G, L, Q, F, W, Y, N, S, E, K, M, T, C, P, H, R, I; preferably, the 233rd amino acid or the 267th amino acid is mutated to R.

In one embodiment, the 328th amino acid is mutated to an amino acid other than E, for example, A, V, G, L, Q, F, W, Y, D, S, K, N, M, T, C, P, H, R, I; preferably, R.

In one embodiment, the 369th amino acid is mutated to an amino acid other than N, for example, A, V, G, L, Q, F, W, Y, D, S, E, K, M, T, C, P, H, R, I; preferably, R.

In one embodiment, the 433rd amino acid is mutated to an amino acid other than S, for example, A, V, G, L, Q, F, W, Y, D, N, E, K, M, T, C, P, H, R, I; preferably, R.

In one embodiment, the 505th amino acid is mutated to an amino acid other than T, for example, A, V, G, L, D, F, W, Y, N, S, Q, E, M, K, C, P, H, R, I; preferably, it is mutated to R.

In one embodiment, compared to SEQ ID No. 3, the amino acid sequence of the parent Cas protein has a sequence identity of at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9%.

In one embodiment, the Cas mutant protein with improved editing activity is selected from any one of the following I-III groups:

I, a Cas mutant protein obtained from a mutation in the amino acid sequence shown in SEQ ID No. 1 at the 7th amino acid site and a mutation at any one or more of the following amino acid sites in the amino acid sequence shown in SEQ ID No. 1: 233rd, 267th, 369th, 433rd, 168th, 328th, and 505th sites;

II, compared to the Cas mutant protein described in I, a Cas mutant protein has the mutation site described in I and has a sequence identity of at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% compared to the Cas mutant protein described in I; and

III, compared to the Cas mutant protein described in I, a sequence has the mutation site described in I and has a substitution, a deletion, or an addition of one or more amino acids compared with the Cas mutant protein described in I; the one or more amino acids include the substitution, the deletion, or the addition of one, two, three, four, five, six, seven, eight, nine, or ten amino acids.

In one embodiment, the Cas mutant protein with improved editing activity is selected from any one of the following I-III groups:

I, a Cas mutant protein by mutating the amino acid sequence shown in SEQ ID No. 3 at any one or more of the following amino acid sites: 233th, 267th, 369th, 433rd, 168th, 328th, and 505th sites; and any one or more of amino acid sites in the Cas mutant protein corresponding to the 7th, 233rd, 267th, 369th, 433rd, 168th, 328th, or 505th site in SEQ ID No. 3 being R;

II, compared to the Cas mutant protein described in I, a Cas mutant protein has the mutation site described in I and has a sequence identity of at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% compared to the Cas mutant protein described in I; and

III, compared to the Cas mutant protein described in I, a sequence has the mutation site described in I and has a substitution, a deletion, or an addition of one or more amino acids compared with the Cas mutant protein described in I; the one or more amino acids include the substitution, the deletion, or the addition of one, two, three, four, five, six, seven, eight, nine, or ten amino acids.

In one embodiment, the amino acid sequence of the parent Cas protein is shown in SEQ ID No. 3.

In some embodiments, the parent Cas protein is a natural wild-type Cas protein; in other embodiments, the parent Cas protein is an engineered Cas protein.

Cas proteins or Cas12i proteins from a variety of organisms can be used as the parent Cas protein, and in some embodiments, the parent Cas protein or the Cas12i protein has nuclease activity. In some embodiments, the parent Cas protein is a nuclease that cuts two strands of a target double-helical nucleic acid (e.g., double-helical DNA). In some embodiments, the parent Cas protein is a nickase that cuts a single strand of the target double-helical nucleic acid (e.g., double-helical DNA).

In one embodiment, the parent Cas protein is a Cas protein of the Cas12 family, preferably a Cas protein of the Cas12i family, for example, Cas 12i 1, Cas12i2, Cas12i3, etc.

In one embodiment, the amino acid sequence of the Cas protein of the Cas12 family, compared to SEQ ID No. 1, has a sequence identity of at least 70%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% or 100%.

In one embodiment, the amino acid sequence of the parent Cas protein, compared to SEQ ID No. 1, has a sequence identity of at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9%.

In this application, it was found that when the above amino acid sites were mutated to positively charged amino acids such as R, H, or K, or to polar uncharged amino acids such as M, F, P, A, W, I, V, and L, the editing activity of the Cas protein could be significantly improved; when mutated to some nonpolar uncharged amino acids such as Q, C, or Y, the editing activity of the Cas protein can also be significantly improved.

It is clear to those skilled in the art that the structure of a protein can be altered without adversely affecting its activity and function, for example, one or more conservative amino acid substitutions can be introduced into the amino acid sequence of a protein without adversely affecting the activity and/or three-dimensional structure of the protein molecule. Those skilled in the art know examples and embodiments of conservative amino acid substitution. Specifically, the amino acid residue can be substituted by another amino acid residue belonging to the same group as the amino acid residue at the site to be substituted, that is, a nonpolar amino acid residue is substituted for another nonpolar amino acid residue, a polar uncharged amino acid residue is substituted for another polar uncharged amino acid residue, a basic amino acid residue is substituted for another basic amino acid residue, and an acidic amino acid residue is substituted for another acidic amino acid residue. Such substituted amino acid residues may or may not be encoded by the genetic code. A conservative substitution of an amino acid by other amino acids belonging to the same group falls within the scope of the invention as long as the substitution does not result in inactivation of the biological activity of protein. Thus, the protein of the invention may include one or more conservative substitutions in the amino acid sequence, which are best produced by substitutions according to Table 1. In addition, the invention also covers proteins that also include one or more other non-conservative substitutions, provided that the non-conservative substitution does not significantly affect the desired function and biological activity of the protein of the invention.

Conservative amino acid replacement can be performed at one or more predicted non-essential amino acid residues. "Non-essential" amino acid residues are amino acid residues that can be altered (absent, substituted, or replaced) without altering biological activity, whereas "essential" amino acid residues are required for biological activity. "Conservative amino acid replacement" is a replacement in which an amino acid residue is replaced by an amino acid residue with a similar side chain. Amino acid replacement can be carried out in non-conserved regions of the above Cas mutant protein. In general, such replacement is not performed on conserved amino acid residues, or on amino acid residues located within conserved moieties, where such residues are required for protein activity. However, those skilled in the art should understand that functional variants can have less conservative or non-conservative variation in conserved regions.

**Table 1**

| Primary residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gin |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gin; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

It is well known that one or more amino acid residues can be altered (replaced, deleted, truncated, or inserted) from the N and/or C terminus of a protein while still preserving its functional activity. Thus, proteins that have altered one or more amino acid residues from the N and/or C terminus of the Cas protein while retaining their required functional activity, are also within the scope of the invention. These alterations may include an alteration introduced by modern molecular methods such as polymerase chain reaction (PCR), and the methods include PCR amplification that alters or lengthens a protein-coding sequence by including an amino acid coding sequence in an oligonucleotide used in PCR amplification.

It should be recognized that proteins can be altered in a variety of ways, including amino acid replacement, deletion, truncation, and insertion, and methods used for such operations are generally known in the field. For example, amino acid sequence variants of the above proteins can be prepared by mutating DNA. It may also be accomplished through other forms of mutagenesis and/or through directed evolution, for example, by using known mutagenesis, recombination, and/or shuffling methods in conjunction with relevant screening methods for substitution, deletion, and/or insertion of single or multiple amino acids.

It is understood by those skilled in the field that these minor amino acid alterations in the Cas protein of the invention can occur (e.g., naturally occurring mutations) or be produced (e.g., using r-DNA technology) without loss of protein function or activity. If these mutations occur in the catalytic domain, active site, or other functional domains of the protein, the nature of the polypeptide may change, but the polypeptide may maintain its activity. If the mutations present are not close to the catalytic domain, active site, or other functional domains, less effect can be expected.

Those skilled in the art can identify the essential amino acids of the Cas mutant protein of the invention on the basis of methods known in the art, such as site-directed mutagenesis, protein evolution, or bioinformatics analysis. The catalytic domain, active site, or other functional domains of a protein can also be determined by physical analysis of the structure, such as by the following techniques: nuclear magnetic resonance, crystallography, electron diffraction, or photoaffinity labeling, combined with presumed amino acid mutations at key sites.

In the present invention, amino acid residues can be represented by a single letter or by three letters, for example: alanine (Ala, A), valine (Val, V), glycine (Gly, G), leucine (Leu, L), glutamine (Gln, Q), phenylalanine (Phe, F), tryptophan (Trp, W), tyrosine (Tyr, Y), aspartic acid (Asp, D), asparagine (Asn, N), glutamic acid (Glu, E), lysine (Lys, K), methionine (Met, M), serine (Ser, S), threonine (Thr, T), cysteine (Cys, C), proline (Pro, P), isoleucine (Ile, 1), histidine (His, H), arginine (Arg, R).

The term "AxxB" represents that amino acid A at xx site is mutated to amino acid B, unless otherwise specified, the amino acid A at xx site from N-terminus is mutated to the amino acid B. For example, S7R represents that S at the 7th site is mutated to R. When multiple amino acid sites have mutations at the same time, it can be expressed in similar forms, such as S7R-Y124R or S7R/Y124R, for example, S7R-Y124R represents that S at the 7th site is mutated to R while Y at the 124th site is mutated to R.

The specific amino acid position (number) in the protein of the invention is determined by aligning the amino acid sequence of the target protein with SEQ ID No. 1 using standard sequence alignment tools, for example, Smith-Waterman algorithm or CLUSTALW2 algorithm are used in two-sequence alignment, where the sequence is considered to be aligned when the alignment score is the highest. The alignment score can be calculated using the method according to Wilbur, W. J. and Lipman, D. J. (1983) Rapid similarity searches ofnucleic acid and protein data banks. Proc. Natl. Acad. Sci. USA, 80:726-730. Default parameters are preferred in ClustalW2(1.82) algorithm: protein gap opening penalty= 10.0; protein gap extension penalty= 0.2; protein matrix= Gonnet; protein/DNA end gap= -1; protein/DNAGAPDIST= 4. It is preferable to adopt the AlignX procedure (part of the vectorNTI group) to fit the default parameters for multiple alignment (gap opening penalty: 10, gap extension penalty: 0.05), to determine the position of a particular amino acid in the protein of the invention by aligning the amino acid sequence of the protein with SEQ ID No. 1.

People in the field can use software commonly used in the field, such as Clustal Omega, to conduct sequence identity comparison and alignment between the amino acid sequence of any parent Cas protein and SEQ ID No. 1 or 3 to obtain the amino acid site in the parent Cas protein corresponding to the amino acid site defined in this application based on SEQ ID No. 1 or 3.

The biological functions of the Cas protein include, but are not limited to, the activity of binding to the guide RNA, the activity of endonuclease, and the activity of binding to and cutting at specific sites of the target sequence under the guidance of the guide RNA, which includes but is not limited to the Cis cleavage activity and Trans cleavage activity.

In the present invention, "Cas mutant protein" may also be referred to as a mutated Cas protein, or a Cas protein variant.

The invention also provides a fusion protein including the above Cas mutant protein and other modification parts.

In one embodiment, the modification part is selected from another protein or polypeptide, a detectable marker, or any combination thereof.

In one embodiment, the modification part is selected from an epitope tag, a reporter gene sequence, a nuclear localization signal (NLS) sequence, a targeting part, a transcriptional activation domain (e.g., VP64), a transcriptional inhibition domain (e.g., KRAB domain or SID domain), a nuclease domain (e.g., Fok1), and domains having activities selected from the following: nucleotide deaminase, cytidine deaminase, adenosine deaminase, methylase activity, demethylase, transcription-activating activity, transcription-inhibiting activity, transcription release factor activity, histone modification activity, nuclease activity, single-stranded RNA cleavage activity, double-stranded RNA cleavage activity, single-stranded DNA cleavage activity, double-stranded DNA cleavage activity, and nucleic acid binding activity; and any combination thereof. The NLS sequence is well known to those skilled in the art, and examples of which include, but are not limited to, SV40 large T antigen, EGL13, c-Myc, and TUS protein.

In one embodiment, the NLS sequence is located at, near, or close to a terminus of the Cas protein of the invention (e.g., N-terminus, C-terminus, or both terminuses).

The epitope tag is well known to those skilled in the art, including, but not limited to, His, V5, FLAG, HA, Myc, VSV-G, Trx, etc., and those skilled in the art may choose other appropriate epitope tags (for example, purification, detection, or tracing).

The reporter gene sequence is well known to those skilled in the art, and examples of which include, but are not limited to, GST, HRP, CAT, GFP, HcRed, DsRed, CFP, YFP, BFP, etc.

In one embodiment, the fusion protein of the invention includes a domain capable of binding to DNA molecules or intracellular molecules, such as maltose binding protein (MBP), DNA binding domain (DBD) of Lex A, DBD of GAL4, etc.

In one embodiment, the fusion protein of the invention includes a detectable marker, such as fluorescent dyes, such as FITC or DAPI.

In one embodiment, the Cas protein of the invention is optionally coupled, conjugated, or fused with the modification part via a linker.

In one embodiment, the modification part is directly connected to either the N-terminus or the C-terminus of the Cas protein of the invention.

In one embodiment, the modification part is connected to the N-terminus or C-terminus of the Cas protein of the invention by means of the linker. Such linkers are well known in the field, and examples of which include, but are not limited to, linkers that include one or more (e.g., one, two, three, four, or five) amino acids (e.g., Glu or Ser) or amino acid derivatives (e.g., Ahx, β-Ala, GABA, or Ava), or PEG, etc.

The Cas protein, protein derivatives, or fusion proteins of the invention are not limited by the way in which they are produced, for example, they may be produced by genetic engineering methods (recombinant technology) or by chemical synthesis methods.

### Nucleic acid of Cas protein

On the other hand, the invention provides an isolated polynucleotide, including:
(a) a polynucleotide sequence encoding the Cas mutant protein or fusion protein of the invention;
   or, a polynucleotide complementary to the polynucleotide described in (a).

In one embodiment, the nucleotide sequence is subjected to codon optimization for expression in prokaryotic cells. In one embodiment, the nucleotide sequence is subjected to codon optimization for expression in eukaryotic cells.

In one embodiment, the cell is an animal cell, for example, a mammalian cell.

In one embodiment, the cell is a human cell.

In one embodiment, the cell is a plant cell, such as cells possessed by cultivated plants (such as cassava, maize, sorghum, wheat, or rice), algae, trees, or vegetables.

In one embodiment, the polynucleotide is preferably single-stranded or double-stranded.

### Guide RNA (gRNA)

On the other hand, the invention provides a gRNA, including a first segment and a second segment; the first segment is also called "skeleton region", "protein binding segment", "protein binding sequence", or "direct repeat sequence"; the second segment is also called "targeting sequence of target nucleic acid" or "targeting segment of target nucleic acid" or "guide sequence for targeting the target sequence".

The first segment of the gRNA is capable of interacting with the Cas protein of the invention so that the Cas protein and the gRNA form a complex.

In the preferred embodiment, the first segment is the direct repeat sequence as described above.

The targeting sequence of the target nucleic acid or the targeting segment of the target nucleic acid of the invention includes a nucleotide sequence that is complementary to the sequence of the target nucleic acid. In other words, the targeting sequence of the target nucleic acid or the targeting segment of the target nucleic acid of the invention is hybridized (i.e., base pairing) to interact with the target nucleic acid in a sequence-specific manner. Thus, the targeting sequence of the target nucleic acid or the targeting segment of the target nucleic acid can be altered or modified to hybridize any desired sequence within the target nucleic acid. The nucleic acid is selected from DNA or RNA.

The complementary percentage between the targeting sequence of the target nucleic acid or the targeting segment of the target nucleic acid and a target sequence of the target nucleic acid may be at least 60% (for example, at least 65%, at least 70%, at least 75%, at least 80%, at least 85%, at least 90%, at least 95%, at least 97%, at least 98%, at least 99%, or 100%).

The "skeleton region", "protein binding segment", "protein binding sequence", or "direct repeat sequence" of the gRNA of the invention can interact with the CRISPR protein (or Cas protein). The gRNA of the invention guides the interacting Cas protein to the specific nucleotide sequence in the target nucleic acid through the action of the targeting sequence of the target nucleic acid.

Preferably, the guide RNA includes the first segment and the second segment from the 5' to 3' direction.

In the present invention, the second segment can also be understood as a guide sequence hybridizing with the target sequence.

The gRNA of the invention is capable of forming a complex with the Cas protein.

### Vector

The invention also provides a vector including the Cas mutant protein, the isolated nucleic acid molecule, or the polynucleotide as described above; preferably, the vector also includes a regulatory element operably linked to it.

In one embodiment, the regulatory element is one or more selected from the group consisting of: enhancer, transposon, promoter, terminator, leader sequence, polyadenylation sequence, and marker gene.

In one embodiment, the vector includes a cloning vector, an expression vector, a shuttle vector, and an integrative vector.

In some embodiments, the vector included in the system is a viral vector (e.g., a retroviral vector, a lentiviral vector, an adenovirus vector, an adeno-associated vector, and a herpes simplex vector), and may also be types such as plasmid, virus, cosmid, phage, etc., which are well known to those skilled in the art.

### CRISPR system

The invention provides an engineered, non-naturally occurring vector system, or a CRISPR-Cas system; the system includes a Cas mutant protein or a nucleic acid sequence encoding the Cas mutant protein and a nucleic acid encoding one or more guide RNA.

In one embodiment, the nucleic acid sequence encoding the Cas mutant protein and the nucleic acid encoding one or more guide RNA are synthesized artificially.

In one embodiment, the nucleic acid sequence encoding the Cas mutant protein and the nucleic acid encoding one or more guide RNA do not co-exist naturally.

The one or more guide RNA target one or more target sequences in the cell. The one or more target sequences hybridize with a genomic locus of the DNA molecule encoding one or more gene products, and guide the Cas protein to the genomic locus of the DNA molecule encoding one or more gene products, and the Cas protein modifies, edits, or cuts the target sequence after reaching the target sequence position. Thus, the expression of one or more of the gene products is altered or modified.

The cell of the invention includes one or more of an animal, plant, or microorganism.

In some embodiments, the Cas protein is codon-optimized for expression in cells.

In some embodiments, the Cas protein guides cleavage of one or two strands at the target sequence location.

The invention also provides an engineered, non-naturally occurring vector system that may include one or more vectors, including:
a) a first regulatory element operably linked to the gRNA, and
b) a second regulatory element operably linked to the Cas protein;
where components (a) and (b) are located on the same or different vectors of the system.

The first and second regulatory elements include a promoter (e.g., a constituent promoter or an inducible promoter), an enhancer (e.g., 35S promoter or 35S enhanced promoter), an internal ribosome entry site (IRES), and other expression control elements (e.g., a transcription termination signal, such as a polyadenylation signal and a polyU sequence).

In some embodiments, the vector included in the system is a viral vector (e.g., a retroviral vector, a lentiviral vector, an adenovirus vector, an adeno-associated vector, and a herpes simplex vector), and may also be types such as plasmid, virus, cosmid, phage, etc., which are well known to those skilled in the art.

In some embodiments, the system presented herein is in a delivery system. In some embodiments, the delivery system is a nanoparticle, a liposome, an exosome, a microvesicle, and a gene gun.

In one embodiment, the target sequence is a DNA or RNA sequence from prokaryotic or eukaryotic cells. In one embodiment, the target sequence is a non-naturally occurring DNA or RNA sequence.

In one embodiment, the target sequence exists within the cell. In one embodiment, the target sequence exists within the nucleus or within the cytoplasm (e.g., organelles). In one embodiment, the cell is a eukaryotic cell. In other embodiments, the cell is a prokaryotic cell.

In one embodiment, the Cas protein is connected to one or more NLS sequences. In one embodiment, the fusion protein includes one or more NLS sequences. In one embodiment, the NLS sequence is connected to the N-terminus or C-terminus of the protein. In one embodiment, the NLS sequence is fused with the N-terminus or C-terminus of the protein.

On the other hand, the invention relates to an engineered CRISPR system including the Cas protein and one or more guide RNA, where the guide RNA includes a direct repeat sequence and a spacer sequence capable of hybridizing with the target nucleic acid, and the Cas protein is capable of binding to the guide RNA and targeting a target nucleic acid sequence that is complementary to the spacer sequence.

### Protein-nucleic acid complex/composition

On the other hand, the present invention provides a complex or composition, including:
(i) a protein component selected from the Cas protein, derived protein, or fusion protein, and any combination thereof; and
(ii) a nucleic acid component including (a) a guide sequence capable of hybridizing with a target sequence; and (b) a direct repeat sequence capable of binding to the Cas protein of the invention.

The protein component combines with the nucleic acid component to form a complex.

In one embodiment, the nucleic acid component is the guide RNA in the CRISPR-Cas system.

In one embodiment, the complex or composition is non-naturally occurring or modified. In one embodiment, at least one component of the complex or composition is non-naturally occurring or modified. In one embodiment, a first component is non-naturally occurring or modified; and/or a second component is non-naturally occurring or modified.

### Activated CRISPR complex

On the other hand, the invention also provides an activated CRISPR complex including: (1) a protein component selected from: the Cas protein, derived protein, or fusion protein of the invention, and any combination thereof; (2) gRNA including (a) a guide sequence capable of hybridizing with a target sequence; and (b) a direct repeat sequence capable of binding to the Cas protein of the invention; and (3) a target sequence bound to the gRNA. Preferably, the binding is a binding of the targeting sequence of the target nucleic acid in the gRNA with the target nucleic acid.

The terms "activated CRISPR complex", "activated complex", or "ternary complex" used in this article refer to the complex formed by the combination or modification of Cas protein, gRNA, and target nucleic acid in the CRISPR system.

The Cas protein and gRNA of the invention can form a binary complex that is activated when bound to a nucleic acid substrate to form the activated CRISPR complex, where the nucleic acid substrate is complementary to the spacer sequence in the gRNA (or the guide sequence for hybridization with the target nucleic acid). In some embodiments, the spacer sequence of the gRNA matches the target substrate exactly. In other embodiments, the spacer sequence of the gRNA matches portions (continuous or discontinuous) of the target substrate.

In the preferred embodiment, the activated CRISPR complex may exhibit collateral nuclease cleavage activity, which refers to the non-specific cleavage activity or random cleavage activity of the activated CRISPR complex on the single-stranded nucleic acid, also known as trans cleavage activity in this field.

### Delivery and delivery composition

The Cas protein, gRNA, fusion protein, nucleic acid molecule, vector, system, complex, and composition of the invention may be delivered by any method known in the art. Such methods include, but are not limited to, electroporation, lipofection, nucleofection, microinjection, sonoporation, gene gun, calcium phosphate mediated transfection, cationic transfection, liposomal transfection, dendritic transfection, heat shock transfection, nucleofection, magnetofection, lipofection, puncture transfection, optical transfection, reagent-enhanced nucleic acid uptake, and delivery via liposomes, immune liposomes, viral particles, artificial virions, etc.

Therefore, on the other hand, the invention provides a delivery composition including a delivery vector and any one or more selected from the following: the Cas protein, fusion protein, nucleic acid molecule, vector, system, complex, and composition of the invention.

In one embodiment, the delivery vector is a particle.

In one embodiment, the delivery vector is selected from a lipid particle, a sugar particle, a metal particle, a protein particle, a liposome, an exosome, a microvesicle, a gene gun, or a viral vector (e.g., replication-deficient retrovirus, lentivirus, adenovirus, or adeno-associated virus).

### Host cell

The invention also relates to an *in vitro, ex vivo,* or *in vivo* cell or cell line or their progeny, which includes the Cas protein, fusion protein, nucleic acid molecule, protein-nucleic acid complex, activated CRISPR complex, vector, and delivery composition of the invention.

In some embodiments, the cell is a prokaryotic cell.

In some embodiments, the cell is a eukaryotic cell. In some embodiments, the cell is a mammalian cell. In some embodiments, the cell is a human cell. In some embodiments, the cell is a non-human mammalian cell, such as those of non-human primates, cattle, sheep, pigs, dogs, monkeys, rabbits, rodents (such as rat or mouse). In some embodiments, the cell is a non-mammalian eukaryotic cell, such as those of poultry birds (such as chicken), fish, or crustaceans (such as clam, shrimp). In some embodiments, the cell is a plant cell, such as those possessed by monocotyledons or dicotyledons or those possessed by cultivated plants or food crops such as cassava, maize, sorghum, soy, wheat, oat, or rice, such as algae, trees, or producing plants, fruits or vegetables (e.g., trees such as citrus tree, nut tree; nightshade, cotton, tobacco, tomato, grape, coffee, cocoa, etc.).

In some embodiments, the cell is a stem cell or a stem cell line.

In some cases, the host cell of the invention includes a genetic or genomic modification that is not present in its wild type.

### Gene editing method and application

The Cas mutant protein, nucleic acid, the composition, the CIRSPR/Cas system, the vector system, the delivery composition, or the activated CRISPR complex, or the host cell of the invention may be used for any one or more of the following purposes: targeting and/or editing a target nucleic acid; cleavage of double-stranded DNA, single-stranded DNA, or single-stranded RNA; non-specific cleavage and/or degradation of collateral nucleic acid; non-specific cleavage of single-stranded nucleic acid; nucleic acid detection; detection of nucleic acid in a target sample; specific editing of double-stranded nucleic acid; base editing of double-stranded nucleic acid; base editing of single-stranded nucleic acid. In other embodiments, it may also be used to prepare a reagent or a kit for any one or more of the above purposes.

The invention also provides an application of the Cas protein, nucleic acid, the composition, the CIRSPR/Cas system, the vector system, the delivery composition, or the activated CRISPR complex in gene editing, gene targeting, or gene cleavage; or in the preparation of a reagent or a kit for gene editing, gene targeting, or gene cleavage.

In one embodiment, the gene editing, gene targeting, or gene cleavage is gene editing, gene targeting, or gene cleavage inside and/or outside the cell.

The invention also provides a method for editing the target nucleic acid, targeting the target nucleic acid, or cutting the target nucleic acid, which includes contacting the target nucleic acid with the Cas protein, nucleic acid, the composition, the CIRSPR/Cas system, the vector system, the delivery composition, or the activated CRISPR complex. In one embodiment, the method is to edit the target nucleic acid, target the target nucleic acid, or cut the target nucleic acid inside or outside the cell.

The gene editing or editing of the target nucleic acid includes modifying the gene, knocking out the gene, altering the expression of the gene product, repairing mutation, and/or inserting the polynucleotide, and gene mutation.

The edits may be made in prokaryotic cells and/or eukaryotic cells.

On the other hand, the invention also provides an application of the Cas protein, nucleic acid, the composition, the CIRSPR/Cas system, the vector system, the delivery composition, or the activated CRISPR complex in nucleic acid detection, or in the preparation of a reagent or a kit for nucleic acid detection.

On the other hand, the invention also provides a method for cutting the single-stranded nucleic acid; the method includes contacting a nucleic acid population with the Cas protein and the gRNA, where the nucleic acid population includes the target nucleic acid and a plurality of non-target single-stranded nucleic acids, and the Cas protein cuts the plurality of non-target single-stranded nucleic acids.

The gRNA is capable of binding to the Cas protein.

The gRNA is capable of targeting the target nucleic acid.

The contact may be inside a cell *in vitro, ex vivo,* or *in vivo.*

Preferably, the cleavage of single-stranded nucleic acid is non-specific cleavage.

On the other hand, the invention also provides an application of the Cas protein, nucleic acid, the composition, the CIRSPR/Cas system, the vector system, the delivery composition, or the activated CRISPR complex in the non-specific cleavage of single-stranded nucleic acid, or in the preparation of a reagent or a kit for the non-specific cleavage of single-stranded nucleic acid.

On the other hand, the invention also provides a kit for gene editing, gene targeting, or gene cleavage, which includes the Cas protein, gRNA, nucleic acid, the composition, the CIRSPR/Cas system, the vector system, the delivery composition, the activated CRISPR complex, or the host cell.

On the other hand, the invention also provides a kit for detecting a target nucleic acid in a sample, which includes: (a) Cas protein, or a nucleic acid encoding the Cas protein; (b) the guide RNA, or a nucleic acid encoding the guide RNA, or a precursor RNA containing the guide RNA, or a nucleic acid encoding the precursor RNA; and (c) a single-stranded nucleic acid detector that does not hybridize with the guide RNA.

Those skilled in the art know that precursor RNA can be cut or processed into the mature guide RNA.

On the other hand, the invention provides a use of the Cas protein, nucleic acid, the composition, the CIRSPR/Cas system, the vector system, the delivery composition, the activated CRISPR complex, or the host cell in the preparation of a preparation or a kit, the preparation or the kit is used for:
(i) gene or genome editing;
(ii) target nucleic acid detection and/or diagnosis;
(iii) editing a target sequence in a target locus to modify biological or non-human organisms;
(iv) treatment of disease; and
(iv) targeting a target gene.

Preferably, the above gene or genome editing is performed within or outside the cell.

Preferably, the target nucleic acid detection and/or diagnosis is performed *in vitro* for target nucleic acid detection and/or diagnosis.

Preferably, the treatment of disease is the treatment of a disease caused by a defect in the target sequence in the target locus.

On the other hand, the invention provides a method for detecting a target nucleic acid in a sample; the method includes contacting the sample with the Cas protein, the gRNA (guide RNA), and the single-stranded nucleic acid detector, the gRNA includes a region bound to the Cas protein and a guide sequence for hybridization with the target nucleic acid; detecting a detectable signal generated by the Cas protein cutting the single-stranded nucleic acid detector, thereby detecting the target nucleic acid; the single-stranded nucleic acid detector is not hybridized with the gRNA.

### Method of specific modification of target nucleic acid

On the other hand, the invention also provides a method of specific modification of a target nucleic acid; the method includes: contacting the target nucleic acid with the Cas protein, the nucleic acid, the composition, the CIRSPR/Cas system, the vector system, the delivery composition, or the activated CRISPR complex.

This specific modification can occur *in vivo or in vitro.*

This specific modification can occur either intracellular or extracellular.

In some cases, the cell is selected from a prokaryotic or eukaryotic cell, for example, an animal cell, a plant cell, or a microbial cell.

In one embodiment, the modification refers to a break in the target sequence, for example, a single/double strand break in DNA, or a single strand break in RNA.

In some cases, the method also includes contacting the target nucleic acid with a donor polynucleotide, where the donor polynucleotide, a portion of the donor polynucleotide, a copy of the donor polynucleotide, or a portion of the copy of the donor polynucleotide is integrated into the target nucleic acid.

In one embodiment, the modification also includes inserting an editing template, such as an exogenous nucleic acid, into the break.

In one embodiment, the method also includes: contacting the editing template with the target nucleic acid or delivering it to a cell containing the target nucleic acid. In the embodiment, the method repairs the broken target gene by homologous recombination with the exogenous template polynucleotide; in some embodiments, the repair causes a mutation that includes the insertion, deletion, or substitution of one or more nucleotides of the target gene, and in other embodiments, the mutation causes an alteration in one or more amino acids in a protein expressed from a gene including the target sequence.

### Detection (non-specific cleavage)

On the other hand, the invention provides a method for detecting a target nucleic acid in a sample; the method includes contacting the sample with the Cas protein, nucleic acid, the composition, the CIRSPR/Cas system, the vector system, the delivery composition, or the activated CRISPR complex and the single-stranded nucleic acid detector; detecting the detectable signal generated by the Cas protein cutting single-stranded nucleic acid detector, thereby detecting the target nucleic acid.

In the present invention, the target nucleic acid includes ribonucleotide or deoxyribonucleotide; including single-stranded nucleic acids and double-stranded nucleic acids, such as single-stranded DNA, double-stranded DNA, single-stranded RNA, and double-stranded RNA.

In one embodiment, the target nucleic acid is derived from a sample of virus, bacterium, microorganism, soil, water source, human body, animal, plant, etc. Preferably, the target nucleic acids are products enriched or amplified by PCR, NASBA, RPA, SDA, LAMP, HAD, NEAR, MDA, RCA, LCR, RAM, and other methods.

In one embodiment, the target nucleic acid is a viral nucleic acid, a bacterial nucleic acid, a disease-related specific nucleic acid, such as a specific mutation site or single nucleotide polymorphism (SNP) site or nucleic acid that differs from control; preferably, the virus is a plant or animal virus, e.g., papillomavirus, hepatic DNA virus, herpesvirus, adenovirus, poxvirus, parvovirus, coronavirus; preferably, the virus is a coronavirus, preferably, SARS, SARS-CoV2 (COVID-19), HCoV-229E, HCoV-OC43, HCoV-NL63, HCoV-HKU1, and Mers-Cov.

In the present invention, the gRNA has a matching degree of at least 50% with the target sequence on the target nucleic acid, preferably at least 60%, preferably at least 70%, preferably at least 80%, preferably at least 90%.

In one embodiment, when the target sequence includes one or more feature sites (such as specific mutation sites or SNP), the feature sites match the gRNA exactly.

In one embodiment, the detection method may include one or more gRNAs with different targeting sequences targeting different target sequences.

In the present invention, the single-stranded nucleic acid detector includes, but is not limited to, single-stranded DNA, single-stranded RNA, a DNA-RNA hybrid, a nucleic acid analog, a base modifier, and a single-stranded nucleic acid detector including a base free spacer, etc.; "nucleic acid analog" includes, but is not limited to, locked nucleic acid, bridged nucleic acid, morpholino nucleic acid, glycol nucleic acid, hexitol nucleic acid, threose nucleic acid, arabinose nucleic acid, 2'-O-methyl RNA, 2'-methoxyacetyl RNA, 2'-fluoro RNA, 2'-amino RNA, 4'-sulfur RNA, and the combination thereof, including optional ribonucleotide or deoxyribonucleotide residues.

In the present invention, the detectable signal is achieved by the following means: vision-based detection, sensor-based detection, color detection, fluorescence signal-based detection, gold nanoparticle-based detection, fluorescence polarization, colloidal phase transition/dispersion, electrochemical detection, and semiconductor-based detection.

In the present invention, preferably, both ends of the single-stranded nucleic acid detector are respectively provided with a fluorophore and a quenching group, and when the single-stranded nucleic acid detector is cut, it can show a detectable fluorescence signal. The fluorophore is selected from any one or more of FAM, FITC, VIC, JOE, TET, CY3, CY5, ROX, Texas Red, or LC RED460; the quenching group is selected from any one or more of BHQ1, BHQ2, BHQ3, Dabcy1, or Tamra.

In other embodiments, the 5' end and the 3' end of the single-stranded nucleic acid detector are respectively provided with different labeling molecules, and the colloidal gold test results of the single-stranded nucleic acid detector before and after being cut by the Cas protein are detected by means of colloidal gold detection; the single-stranded nucleic acid detector will show different color rendering results on the detection line and quality control line of colloidal gold before and after being cut by the Cas protein.

In some embodiments, the method for detecting target nucleic acid may also include comparing a level of the detectable signal with a level of the reference signal and determining an amount of the target nucleic acid in the sample based on the level of the detectable signal.

In some embodiments, the method for detecting target nucleic acid may also include using a RNA reporter nucleic acid and a DNA reporter nucleic acid (e.g., fluorescence color) on different channels, determining the level of the detectable signal by measuring signal levels of RNA and DNA reporter molecules and by measuring the amount of target nucleic acids in RNA and DNA reporter molecules, and sampling based on the level of detectable signal in a combination (for example, using a minimum or product).

In one embodiment, the target gene exists within the cell.

In one embodiment, the cell is a prokaryotic cell.

In one embodiment, the cell is a eukaryotic cell.

In one embodiment, the cell is an animal cell.

In one embodiment, the cell is a human cell.

In one embodiment, the cell is a plant cell, such as those possessed by cultivated plants (such as cassava, maize, sorghum, wheat, or rice), algae, trees, or vegetables.

In one embodiment, the target gene is present in a nucleic acid molecule (e.g., plasmid) *in vitro.*

In one embodiment, the target gene is present in a plasmid.

### Term definition

In the present invention, unless otherwise stated, the scientific and technical terms used herein have meanings commonly understood by those skilled in the art. In addition, the procedures used in this article, such as molecular genetics, nucleic acid chemistry, chemistry, molecular biology, biochemistry, cell culture, microbiology, cell biology, genomics, and recombinant DNA, are common procedures widely used in the corresponding field. At the same time, in order to better understand the invention, definitions and explanations of relevant terms are provided below.

Nucleic acid cleavage or cutting nucleic acid in this article includes: DNA or RNA break in target nucleic acid produced by Cas enzyme described herein (Cis cleavage), DNA or RNA break in collateral nucleic acid substrate (single-stranded nucleic acid substrate) (i.e., non-specific or non-targeted, Trans cleavage). In some embodiments, the cleavage is a double-stranded DNA break. In some embodiments, the cleavage is a single-stranded DNA break or a single-stranded RNA break.

### CRISPR system

As used herein, the terms "clustered regularly interspaced short palindromic repeats (CRISPR)-CRISPR-associated (Cas) (CRISPR-Cas) system" or "CRISPR system" are used commutatively and have a meaning commonly understood by those skilled in the art; it usually includes a transcription product or other elements that are related to the expression of CRISPR-associated ("Cas") gene, or a transcription product or other elements capable of guiding the activity of the Cas gene.

### CRISPR/Cas complex

As used herein, the term "CRISPR/Cas complex" refers to a complex formed by the binding of a guide RNA or a mature crRNA to a Cas protein, which includes a direct repeat sequence hybridized to a guide sequence of a target sequence and bound to the Cas protein; the complex is able to recognize and cut polynucleotides that can hybridize with the guide RNA or the mature crRNA.

### Guide RNA (gRNA)

As used herein, the terms "guide RNA (gRNA)", "mature crRNA", and "guide sequence" are used commutatively and have a meaning commonly understood by those skilled in the art. In general, a guide RNA can include a direct repeat sequence and a guide sequence or be substantially composed of or composed of the direct repeat sequence and the guide sequence.

In some cases, the guide sequence is any polynucleotide sequence that is sufficiently complementary to the target sequence to hybridize with the target sequence and guide the specific binding of the CRISPR/Cas complex to the target sequence. In one embodiment, when it is the best alignment, the degree of complementarity between the guide sequence and its corresponding target sequence is at least 50%, at least 60%, at least 70%, at least 80%, at least 90%, at least 95%, or at least 99%. Determine the best alignment within the competence of a general skilled person in the field. For example, there are publicly available and commercially available algorithms and programs for alignment, such as but not limited to ClustalW, Smith-Waterman in matlab, Bowtie, Geneious, Biopython, and SeqMan.

### Target sequence

"Target sequence" refers to a polynucleotide targeted by a guide sequence in a gRNA, such as a sequence that is complementary to the guide sequence, where hybridization between the target sequence and the guide sequence will facilitate the formation of a CRISPR/Cas complex (including Cas protein and gRNA). Complete complementarity is not necessary as long as there is sufficient complementarity to cause hybridization and facilitate the formation of the CRISPR/Cas complex.

The target sequence can include any polynucleotide, such as DNA or RNA. In some cases, the target sequence is located inside or outside the cell. In some cases, the target sequence is located in the nucleus or cytoplasm of the cell. In some cases, the target sequence may be located in an organelle of eukaryotic cells such as the mitochondria or chloroplast. A sequence or template that can be used to reassemble into a target locus that includes the target sequence is called an "editing template" or "editing polynucleotide" or "editing sequence". In one embodiment, the editing template is an exogenous nucleic acid. In one embodiment, the recombination is homologous recombination.

In the present invention, "target sequence" or "target polynucleotide" or "target nucleic acid" may be any endogenous or exogenous polynucleotide to a cell (for example, eukaryotic cell). For example, the target polynucleotide may be a polynucleotide present in the nucleus of a eukaryotic cell. The target polynucleotide can be a sequence encoding a gene product (for example, protein) or a non-coding sequence (for example, regulatory polynucleotide or useless DNA). In some cases, this target sequence should be related to the protospacer adjacent motif (PAM).

### Single-stranded nucleic acid detector

The single-stranded nucleic acid detector of the invention refers to a sequence including 2-200 nucleotides, preferably having 2-150 nucleotides, preferably, 3-100 nucleotides, preferably, 3-30 nucleotides, preferably, 4-20 nucleotides, and more preferably, 5-15 nucleotides. It is preferred to be a single-stranded DNA molecule, a single-stranded RNA molecule, or a single-stranded DNA-RNA hybrid.

The two ends of the single-stranded nucleic acid detector include different reporter groups or labeling molecules; when it is in an initial state (that is, not cut), it does not present a report signal, and when the single-stranded nucleic acid detector is cut, it presents a detectable signal, that is, it shows a detectable difference after cutting and before cutting.

In one embodiment, the reporter group or labeling molecule includes a fluorophore and a quenching group; the fluorophore is selected from any one or more of FAM, FITC, VIC, JOE, TET, CY3, CY5, ROX, Texas Red, or LC RED460; the quenching group is selected from any one or more of BHQ1, BHQ2, BHQ3, Dabcy1, or Tamra.

In one embodiment, the single-stranded nucleic acid detector has a first molecule connected to the 5 'end (such as FAM or FITC) and a second molecule connected to the 3' end (such as biotin). The reaction system including the single-stranded nucleic acid detector cooperates with a flow strip to detect the target nucleic acid (preferably, colloidal gold detection method). The flow strip is designed to have two capture lines, with an antibody that binds to the first molecule (i.e., a first molecule antibody) at the sample contact end (colloidal gold), an antibody that binds to the first molecule antibody at the first line (control line), and an antibody to the second molecule that binds to the second molecule at the second line (test line) (i.e., a second molecule antibody, such as avidin). As the reaction flows along the strip, the first molecule antibody binds to the first molecule and carries the cut or uncut oligonucleotides to the capture line, and the cut reporter will bind the antibody of the first molecule antibody at the first capture line, while the uncut reporter will bind the second molecule antibody at the second capture line. The combination of the reporter groups in each line will result in a strong readout/signal (e.g., color). As more reporters are cut, more signals will accumulate at the first capture line, and fewer signals will appear at the second line. In some respects, the present invention relates to the use of the flow strip for detecting nucleic acid, as described herein. In some respects, the present invention relates to a method of detecting nucleic acid with the flow strip defined herein, such as (lateral) flow testing or (lateral) flow immunochromatography determination. In some respects, the molecules in the single-stranded nucleic acid detector can be replaced with each other, or the position of the molecules can be changed, provided that the reporting principle is the same or similar to the invention, and the improved manner is also included in the invention.

The detection method of the invention can be used for a quantitative detection of a target nucleic acid to be detected. The quantitative detection index can be quantified according to the signal strength of the reporter group, such as according to the luminous intensity of the fluorophore, or according to the width of the color rendering band.

### Wild type

As used herein, the term "wild type" has a meaning commonly understood by those skilled in the art, which represents a characteristic of an organism, strain, or gene that is typical of it or that distinguishes it from a mutant or variant form when it exists in nature, is separable from a natural source, and has not been intentionally modified by humans.

### Derivatization

As used herein, the term "derivatization" means a chemical modification of an amino acid, polypeptide, or protein to which one or more substituents have been covalently linked. Substituents can also be called side chains.

A derived protein is a derivative of the protein, and in general, the derivatization of the protein does not adversely affect the desired activity of the protein (for example, binding activity to the guide RNA, endonuclease activity, or the activity of binding to and cutting at a specific site of the target sequence under the guidance of the guide RNA); that is, the derivative of the protein has the same activity as the protein.

### Derived protein

Also known as "protein derivative", refers to a modified form of a protein in which, for example, one or more amino acids of the protein can be deleted, inserted, modified, and/or substituted.

### Non-naturally occurring

As used herein, the terms "non-naturally occurring" or "engineered" are used commutatively and indicate artificial involvement. When these terms are used to describe nucleic acid molecules or polypeptides, they mean that the nucleic acid molecules or polypeptides are at least basically free from at least one other component which they are present in nature or to which they are bound if found in nature.

### Orthologue, ortholog

As used herein, the term "orthologue, ortholog" has a meaning commonly understood by those skilled in the art. As a further guide, an "orthologue, ortholog" of a protein, as described herein, refers to a protein belonging to a different species that performs the same or similar functions as a protein that is its ortholog.

### Identity

As used herein, the term "identity" is used to refer to the matching condition of sequences between two polypeptides or between two nucleic acids. When a position in two sequences being compared is occupied by the same base or amino acid monomer subunit (for example, a position in each of two DNA molecules is occupied by adenine, or a position in each of two polypeptides is occupied by lysine), then the molecules are identical at that position. The "percent identity" between two sequences is a function of the number of matching positions shared by the two sequences divided by the number of positions being compared × 100. For example, if 6 out of 10 positions of two sequences match, then the two sequences have an identity of 60%. For example, the DNA sequences CTGACT and CAGGTT share an identity of 50% (matching 3 out of 6 positions in total). Typically, a comparison is made when two sequences are aligned to produce maximum identity. Such a comparison can be achieved by using, for example, a computer program such as the Align program (DNAstar, Inc.) to expediently conduct the method from Needleman et al. (1970) J. Mol. Biol. 48: 443-453. The E. Meyers and W. Miller (Comput. Appl Biosci., 4:11-17 (1988)) algorithm that has been integrated into the ALIGN program (version 2.0), and the PAM120 weight residue table, the 12 gap length penalty, and the 4 gap penalty can be used to determine the percent identity between two amino acid sequences. In addition, the Needleman and Wunsch (J MoI Biol. 48:444-453 (1970)) algorithm that has been integrated into the GAP program of the GCG package (available at www.gcg.com), and the Blossum 62 matrix or PAM250 matrix, the gap weight of 16, 14, 12, 10, 8, 6, or 4, and the length weight of 1, 2, 3, 4, 5, or 6 can be used to determine the percent identity between two amino acid sequences.

### Vector

The term "vector" refers to a nucleic acid molecule that is capable of transporting another nucleic acid molecule connected to it. The vector includes, but is not limited to, a single-stranded, double-stranded, or partially double-stranded nucleic acid molecule; a nucleic acid molecule including one or more free ends and no free ends (e.g., circular); a nucleic acid molecule including DNA, RNA, or both; and a variety of other polynucleotides known in the field. The vector can be introduced into a host cell by transformation, transduction, or transfection so that the genetic material elements carried by it can be expressed in the host cell. The vector can be introduced into a host cell to produce a transcript, protein, or peptide, including the proteins, fusion proteins, isolated nucleic acid molecules, etc., as described herein (e.g., CRISPR transcripts, such as nucleic acid transcripts, proteins, or enzymes). The vector may include a variety of elements that control expression, including, but not limited to, a promoter sequence, a transcription initiation sequence, an enhancer sequence, a selection element, and a reporter gene. In addition, the vector may include a replication initiation site.

One type of vector is "plasmid", which refers to a circular double-stranded DNA ring in which additional DNA fragments can be inserted, for example, by standard molecular cloning techniques.

Another type of vector is a viral vector, in which virus-derived DNA or RNA sequences are present in a vector used to package virus (e.g., retrovirus, replication-deficient retrovirus, adenovirus, replication-deficient adenovirus, and adeno-associated virus). The viral vector also includes a polynucleotide carried by the virus for transfection into one type of host cell. Certain vectors (e.g., a bacterial vector with a bacterial replication starting point and an episomal mammalian vector) are able to replicate autonomously in the host cell into which they are introduced.

Other vectors (e.g., non-episomal mammalian vector) integrate into the genome of a host cell after introduction, and thus replicate with the host genome. Moreover, certain vectors are able to guide the expression of genes that can be operably linked to them. Such vectors are called "expression vector" here.

### Host cell

As used herein, the term "host cell" refers to a cell that may be used to introduce a vector, including, but not limited to, prokaryotic cells, such as *Escherichia coli* or *Bacillus subtilis,* and eukaryotic cells, such as microbial cells, fungal cells, animal cells, and plant cells.

Those skilled in the art will understand that the design of an expression vector can depend on factors such as the selection of host cells to be transformed, the desired level of expression, etc.

### Regulatory element

As used herein, the term "regulatory element" is intended to include a promoter, an enhancer, an internal ribosome entry site (IRES), and other expression control elements (such as a transcription termination signal, such as a polyadenylation signal and a polyU sequence), for which a detailed description can be found in Goeddel, "GENE EXPRESSION TECHNOLOGY: METHODS IN ENZYMOLOGY" 185, Academic Press, San Diego, California (1990). In some cases, the regulatory element includes those sequences that guide constitutive expression of a nucleotide sequence in many types of host cells and those sequences that guide expression of the nucleotide sequence only in certain host cells (e.g., tissue-specific regulatory sequence). A tissue-specific promoter may primarily guide expression in a desired tissue of interest, such as muscle, neuron, bone, skin, blood, a specific organ (e.g., liver, pancreas), or a specific cell type (e.g., lymphocyte). In some cases, the regulatory element may also guide expression in a time-dependent manner (e.g., in a cell cycle-dependent or developmental stage-dependent manner) that may or may not be tissue-specific or cell type-specific. In some cases, the term "regulatory element" covers enhancer elements, such as WPRE; CMV enhancer; the R-U5' fragment in the LTR of HTLV-I (Mol. Cell. Biol., Vol. 8(1), pp. 466-472, 1988); SV40 enhancer; and the intron sequence between exons 2 and 3 of rabbit β-globin (Proc. Natl. Acad. Sci. USA., vol. 78(3), pp. 1527-31, 1981).

### Promoter

As used herein, the term "promoter" has a meaning known to those skilled in the art and refers to a non-coding nucleotide sequence located upstream of a gene that initiates downstream gene expression. A constitutive promoter is a nucleotide sequence that, when operably linked to a polynucleotide encoding or defining a gene product, leads to the production of the gene product in a cell under most or all physiological conditions. An inducible promoter is a nucleotide sequence that, when operably linked to a polynucleotide encoding or defining a gene product, results in the production of the gene product in a cell basically only if an inducer corresponding to the promoter is present in the cell. A tissue-specific promoter is a nucleotide sequence that, when operably linked to a polynucleotide encoding or defining a gene product, results in the production of the gene product in a cell basically only if the cell is a cell of the tissue type to which the promoter corresponds.

### NLS

"Nuclear localization signal" or "nuclear localization sequence" (NLS) is an amino acid sequence that "tags" a protein for introduction into the nucleus by nuclear transport, i.e., proteins with NLS are transported to the nucleus. Typically, NLS includes positively charged Lys or Arg residues that are exposed on the surface of the protein. Exemplary nuclear localization sequences include, but are not limited to, NLS from the following: SV40 large T antigen, EGL-13, c-Myc, and TUS protein. In some embodiments, the NLS includes a PKKKRKV sequence. In some embodiments, the NLS includes an AVKRPAATKKAGQAKKKKLD sequence. In some embodiments, the NLS includes a PAAKRVKLD sequence. In some embodiments, the NLS includes an MSRRRKANPTKLSENAKKLAKEVEN sequence. In some embodiments, the NLS includes a KLKIKRPVK sequence. Other nuclear localization sequences include, but are not limited to, the acidic M9 domain of hnRNP A1, the KIPIK and PY-NLS sequences in yeast transcription repressor Matα2.

### Operably linked

As used herein, the term "operably linked" is intended to indicate that a nucleotide sequence of interest is linked to one or more regulatory elements in a manner that allows the expression of the nucleotide sequence (for example, in an *in vitro* transcription/translation system or in a host cell when the vector is introduced into the host cell).

### Complementarity

As used herein, the term "complementarity" refers to the ability of a nucleic acid to form one or more hydrogen bonds with another nucleic acid sequence by means of traditional Walson-Crick or other non-traditional types. The complementary percentage represents the percentage of residues in a nucleic acid molecule that can form hydrogen bonds with a second nucleic acid sequence (e.g., Watson-Crick base pairing) (e.g., 5, 6, 7, 8, 9, and 10 out of 10 are 50%, 60%, 70%, 80%, 90%, and 100% complementary). "Complete complementarity" means that all continuous residues of a nucleic acid sequence form hydrogen bonds with the same number of continuous residues in a second nucleic acid sequence. As used herein, "substantially complementary" means a region including 8, 9, 10, 11, 12, 13, 14, 15, 16, 17, 18, 19, 20, 21, 22, 23, 24, 25, 30, 35, 40, 45, 50, or more nucleotides has a degree of complementarity of at least 60%, 65%, 70%, 75%, 80%, 85%, 90%, 95%, 97%, 98%, 99%, or 100%, or refers to two nucleic acids that are hybridized under a strict condition.

### Strict condition

As used herein, a "strict condition" for hybridization is a condition in which a nucleic acid that is complementary to a target sequence hybridizes primarily with the target sequence and substantially does not hybridize to a non-target sequence. The strict condition is usually sequence-dependent and varies depending on many factors. In general, the longer the sequence, the higher the temperature at which the sequence specifically hybridizes to its target sequence.

### Hybridization

The term "hybridization" or "complementary" or "substantially complementary" refers to the fact that a nucleic acid (e.g., RNA, DNA) includes a nucleotide sequence that enables it to bind non-covalently, i.e., to form base pairs and/or G/U base pairs with another nucleic acid in a sequence-specific, antiparallel manner (i.e., nucleic acids specifically bind complementary nucleic acids), "annealing" or "hybridization".

Hybridization requires two nucleic acids to contain complementary sequences, although there may be mispairing between bases. The suitable conditions for hybridization between two nucleic acids depend on the length and degree of complementarity of the nucleic acids, which are well-known variables in the field. Typically, the length of a nucleic acid that can be hybridized is 8 nucleotides or more (for example, 10 nucleotides or more, 12 nucleotides or more, 15 nucleotides or more, 20 nucleotides or more, 22 nucleotides or more, 25 nucleotides or more, or 30 nucleotides or more).

It should be understood that the sequence of a polynucleotide does not need to be 100% complementary to the sequence of its target nucleic acid for specific hybridization. The polynucleotide sequence may have a complementarity of 60% or higher, 65% or higher, 70% or higher, 75% or higher, 80% or higher, 85% or higher, 90% or higher, 95% or higher, 98% or higher, 99% or higher, 99.5% or higher, or 100% with a sequence of the target region in the target nucleic acid hybridized with it.

Hybridization of target sequence with gRNA represents that at least 60%, 70%, 75%, 80%, 85%, 90%, 91%, 92%, 93%, 94%, 95%, 96%, 97%, 98%, 99%, or 100% of the target sequence and the nucleic acid sequence of gRNA can be hybridized to form a complex; or represents at least 12, 15, 16, 17, 18, 19, 20, 21, 22, or more bases of the target sequence and the nucleic acid sequence of gRNA can be complementary paired and hybridized to form a complex.

### Expression

As used herein, the term "expression" refers to a process whereby a DNA template is transcribed into a polynucleotide (e.g., into mRNA or other RNA transcripts) and/or a process whereby the transcribed mRNA is subsequently translated into a peptide, polypeptide, or protein. Transcripts and encoded polypeptides can be collectively referred to as the "gene product". If the polynucleotide is derived from genomic DNA, expression can include splicing of mRNA in eukaryotic cells.

### Linker

As used herein, the term "linker" refers to a linear polypeptide formed from multiple amino acid residues connected by peptide bonds. The linker of the invention may be a synthetic amino acid sequence or a naturally occurring polypeptide sequence, such as a polypeptide having a hinge region function. Such linker polypeptides are well known in the field (refer to e.g., Holliger, P. et al. (1993) Proc. Natl. Acad. Sci. USA 90:6444-6448; Poljak, R. J. et al. (1994) Structure 2:1121-1123).

### Treatment

As used herein, the term "treatment" means treating or curing a disease, delaying the onset of symptoms of a disease, and/or delaying the progression of a disease.

### Subject

As used herein, the term "subject" includes, but is not limited to, various animals, plants, and microorganisms.

### Animal

For example, mammals, such as animals of Bovidae, Equidae, Caprinae, Suidae, Canidae, Felidae, and Leporidae, rodents (e.g., mouse or rat), non-human primates (e.g., macaque or crab-eating monkey), or humans. In some embodiments, the subject (e.g., human) has a disease (e.g., a disease caused by a defect in a disease-related gene).

### Plant

The term "plant" should be understood to mean any differentiated multicellular organism capable of photosynthesis, including crop plants at any stage of maturity or development, especially monocotyledons or dicotyledons, vegetable crops, including artichoke, kohlrabi, arugula, leek, asparagus, lettuce (e.g., head lettuce, leaf lettuce, romaine lettuce), bokchoy, malanga, melons (e.g., melon, watermelon, crenshaw, honeydew, cantaloupe), rape crops (e.g., brussels sprouts, cabbage, cauliflower, broccoli, curly kale, kale, Chinese cabbage, bokchoy), cardoon, carrot, napa, okra, onion, celery, parsley, chickpea, parsnip, chicory, pepper, potato, cucurbit (e.g., baby marrow, cucumber, zucchini, cushaw, pumpkin), radish, dry bulb onion, rutabaga, purple eggplant (also known as eggplant), oyster plant, sonchus brachyotus, shallot, endive, garlic, spinach, green onion, cushaw, greens, beet (sugar beet and mangel), sweet potato, swiss chard, wasabi, tomato, turnip, and spice; fruits and/or vine crops, such as apple, apricot, cherry, nectarine, peach, pear, plum, prune, cherry, quince, almond, chestnut, hazelnut, pecan, pistachio, walnut, citrus, blueberry, boysenberry, cranberry, ribe nigrum, loganberry, raspberry, strawberry, blackberry, grape, avocado, banana, kiwi, persimmon, pomegranate, pineapple, tropical fruit, pome, melon, mango, papaya, and lychee; field crops, such as clover, alfalfa, evening primrose, meadowfoam, corn/maize (field corn, sweet corn, popcorn), hops, jojoba, peanut, rice, safflower, small grain cereal crops (barley, oat, rye, wheat, etc.), sorghum, tobacco, kapok, legumes (beans, lentil, pea, soybean), oil plants (oilseed rape, mustard, poppy, olive, sunflower, coconut, castor oil plants, cocoa bean, groundnut), Arabidopsis, fiber plants (cotton, flax, hemp, jute), Lauraceae (cinnamon, camphor), or a plant such as coffee, sugar cane, tea, and natural rubber plants; and/or bedding plants, such as flowering plants, cactus, succulents and/or ornamentals, and trees, such as forests (broad-leaved trees and evergreen trees, such as coniferous tree), fruit trees, ornamental trees, and nut-bearing trees, as well as shrubs and other seedlings.

### Beneficial effects of invention

The invention improves the activity of Cas12i3 protein by mutation and has broad application prospects.

The embodiments of the invention are described in detail below in conjunction with the drawings and embodiments, but those skilled in the art will understand that the following drawings and embodiments are used only to illustrate the invention and not to limit the scope of the invention. The various purposes and advantages of the present invention will become apparent to those skilled in the art according to the following detailed description of the drawings and preferred embodiments.

### DESCRIPTION OF THE DRAWINGS

FIG. 1. Verification of editing efficiency of Cas protein with amino acid mutation at different single sites in the cell.
FIG. 2. Verification of editing efficiency of Cas protein with different mutations at the 7th amino acid site in the cell, with S7S as the wild-type control.
FIG. 3. Verification of editing efficiency of mutant Cas protein at different target locations.
FIG. 4. Validation of trans activity of mutant Cas protein *in vitro.*
FIG. 5. Schematic diagram of Cas-GFFP-mCherry vector; where A is the vector diagram and B is the GFFP structure diagram.
FIG. 6. Verification of editing efficiency of Cas protein with different amino acid mutations in the cell.
FIG. 7. Editing efficiency of different Cas proteins in CHO cells.
FIG. 8. Editing efficiency of different Cas proteins in 293T cells.

### SPECIFIC IMPLEMENTATIONS

The following examples are used only to describe, and not to limit, the invention. Unless otherwise specified, the experiments and methods described in the examples are carried out substantially in accordance with conventional methods well known in the field and described in various references. For example, the conventional techniques of immunology, biochemistry, chemistry, molecular biology, microbiology, cell biology, genomics, and recombinant DNA used in the invention can be found in Sambrook, Fritsch, and Maniatis, "MOLECULAR CLONING: A LABORATORY MANUAL", second editor (1989); "CURRENT PROTOCOLS IN MOLECULAR BIOLOGY" F. M. Ausubel et al., eds. (1987); "METHODS IN ENZYMOLOGY" series (academic publishing company): "PCR 2: A PRACTICAL APPROACH" (M. j. Macpherson, B. D. Hames and G. R. Taylor, eds. (1995)) and Harlow and Lane, eds. (1988) "ANTIBODIES, A LABORATORY MANUAL", and "ANIMAL CELL CULTURE" (R. I. Fleshney, eds. (1987)).

In addition, where the specific conditions are not indicated in the examples, they shall be carried out in accordance with the usual conditions or those recommended by the manufacturer. The reagents or instruments used, where the manufacturer is not indicated, are conventional products that can be obtained through market purchase. Those skilled in the art know that examples describe the invention by way of example and are not intended to limit the scope of protection required by the invention. All disclosures and other references referred to herein are incorporated by reference in their entirety.

### Example 1. Acquisition of Cas mutant protein

For the known Cas protein (Cas12f.4 in CN111757889B, referred to as Cas12i3 in this example), the applicant predicted the key amino acid site that may affect its biological function through bioinformatics, mutated the amino acid site, and obtained a Cas mutant protein with improved editing activity. Specifically, the coding sequence of Cas12i3 was codon optimized (human) and synthesized; the amino acid sequence of wild-type Cas12i3 is shown in SEQ ID No. 1, and its nucleic acid sequence is shown in SEQ ID No. 2, site-directed mutagenesis of amino acids in Cas12i3 potentially bound to the target sequence was carried out by bioinformatics methods.

Variants of the Cas protein were generated by PCR-based site-directed mutagenesis. The specific method is to divide the DNA sequence of the Cas12i3 protein into two parts with the mutation site as the center, design two pairs of primers to amplify the two parts of the DNA sequence, and introduce the sequence that needs to be mutated into the primers, finally, load the two fragments onto the pcDNA3.3-eGFP vector by Gibson clone. The combination of mutants was constructed by splitting the DNA of the Cas12i3 protein into multiple segments using PCR and Gibson clone. Fragment Amplification Kit: TransStart FastPfu DNA Polymerase (including 2.5 mM dNTPs), the specific experimental process is detailed in the manual. Gel Extraction Kit: ^{FastPure®}Gel DNA Extraction Mini Kit, the specific experimental process is detailed in the manual. Kit used for vector construction: pEASY-Basic Seamless Cloning and Assembly Kit (CU201-03), the specific experimental process is detailed in the manual. The mutant amino acid sites involved and the primers used are shown in the following table:

| Primer name | Primer sequence (underline is the mutation site) | Amino acid mutation type (amino acid site from the N-terminus of SEQ ID No. 1) |
|---|---|---|
| I3-S7R-F | AGGAGACCCTACCAGTCCCTCCTCCTGCCCAAC | S7R |
| I3-S7R-R | GACTGGTAGGGTCTCCTCACCTCCACCTTCTTC | |
| I3-P9R-F | AGGTACCAGTCCCTCCTCCTGCCCAACCACAG | P9R |
| I3-P9R-R | AGGAGGGACTGGTACCTTCTGGACACCTCCACC | |
| I3-Q11R-F | TGTCCAGACCCTACAGGTCCCTCCTCCTGCCCAAC | Q11R |
| I3-Q11R-R | CCTGTAGGGTCTGGACACCTCCACCTTCTTCTTATCG | |
| I3-Y124R-F | AGGATGTGGATCGACTGCGCCTGGGAGGCCGATAG | Y124R |
| I3-Y124R-R | CAGTCGATCCACATCCTCTTCTTGGGGTCGAAGTTG | |
| I3-T354R-F | AGGCCTGACAAGTTCGTGATCAAGCCAGAGCACATC | T354R |
| I3-T354R-R | ACGAACTTGTCAGGCCTCTTGTGGAACTCAGAG | |
| I3-P355R-F | AGGGACAAGTTCGTGATCAAGCCAGAGCACATCG | P355R |
| I3-P355R-R | ATCACGAACTTGTCCCTTGTCTTGTGGAACTCAGAG | |

Based on the above amino acid mutation sites, the wild-type protein (WT) of Cas12i3 and the proteins with a mutation at the single amino acid site (named after the mutation type) were obtained, respectively: S7R, P9R, Q11R, Y124R, T354R, and P355R. Relative to the sequence shown in SEQ ID No. 1, the 7th, 9th, 11th, 124th, 354th, and 355th site amino acids from the N-terminus of the proteins with mutation are mutated to R, respectively.

### Example 2. Verification of editing activity of Cas mutant protein

Different Cas proteins obtained in Example 1 were used to verify their gene editing activity in animal cells, and a target was designed for Chinese hamster ovary cell (CHO) FUT8 gene, FUT8-Cas-XX-g3: *TTC*CAGCCAAGGTTGT GGACGGATCA, the italic part is the PAM sequence, and the underlined area is the target region. The vector pcDNA3.3 was modified to carry EGFP fluorescent protein and PuroR resistance gene. The SV40NLS-Cas-XX fusion protein was inserted through enzyme cutting sites XbaI and PstI; the U6 promoter and gRNA sequence were inserted through enzyme cutting site Mfe1. The CMV promoter activated the expression of fusion protein SV40NLS-Cas-XX-NLS-GFP. The protein Cas-XX-NLS was linked to the protein GFP by the linker peptide T2A. Promoter EF-1α activated the expression of the puromycin resistance gene. Seeding: seeding was conducted when the CHO cell confluency was 70-80%, and the number of inoculated cells in the 12-well plate was 8×10⁴ cells/well. Transfection: transfection was conducted 24 h after seeding, 6.25 µL Hieff Trans^{™} liposome nucleic acid transfection reagent was added to 100 µL opti-MEM and mixed well; 2.5 ug plasmid was added to 100 µL opti-MEM and mixed well. The diluted Hieff Trans^{™} liposome nucleic acid transfection reagent was mixed well with the diluted plasmid and incubated at room temperature for 20 min. The incubated mixture was added to a medium with seeded cells for transfection. Screening with puromycin: puromycin was added 24 h after transfection, the final concentration was 10 µg/mL. After treatment with puromycin for 24 h, the culture medium was changed into normal medium, and the culture lasted for 24 h. 48 h after transfection, the cells were digested with trypsin-EDTA (0.05%), and the cells with a GFP signal were sorted by flow cytometry (fluorescence-activated cell sorting (FACS)).

DNA extraction, PCR amplification near the editing area, sent to hiTOM sequencing: the cells were digested by pancreatic enzyme and collected, and the genomic DNA was extracted by a cell/tissue genomic DNA extraction kit (Bioteke). The region near the target of genomic DNA was amplified. PCR products were sequenced by hiTOM. Sequencing data were analyzed, sequence types and proportions within the range of 15 nt upstream and 10 nt downstream of target position were counted, and sequences with single nucleotide variant (SNV) frequency greater than/equal to 1% or non-SNV mutation frequency greater than/equal to 0.06% were counted, to obtain the editing efficiency of Cas-XX protein on target position. CHO cell FUT8 target gene sequence: FUT8-Cas-XX-g3: *TTC*CAGCCAAGGTTGTGGACGGATCA, the italic part is the PAM sequence, and the underlined area is the target region. The gRNA sequence is: AGAGAAUGUGUGCAUAGUCAACAC CAGCCAAGGUUGUGGACGGAUCA, the underlined area is the target region, other areas are DR (direct repeat sequence) regions.

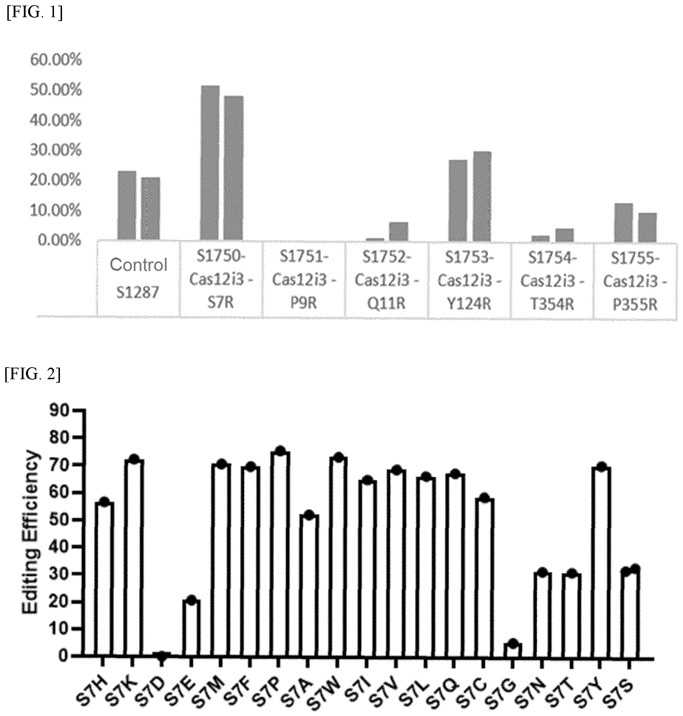

FIG. 1 shows the editing activity of wild-type Cas12i3 protein (WT) and mutant proteins mutated at a single amino acid site. As shown in FIG. 1, the control group was wild type, and the vector number was S1287; after the mutation of the 7th amino acid, the vector was S1750-Cas12i3-S7R; after the mutation of the 9th amino acid, the vector was S1751-Cas12i3-P9R; after the mutation of the 11th amino acid, the vector was S1752-Cas12i3-Q11R.; after the mutation of the 124th amino acid, the vector was S1753-Cas12i3-Y124R; after the mutation of the 354th amino acid, the vector was S1754-Cas12i3-T354R; after the mutation of the 355th amino acid, the vector was S1755-Cas12i3-P355R, and two repeats were performed for each site: repeat 1 and repeat 2. The editing efficiency of mutant protein was verified in CHO cells. The editing efficiencies of repeat 1 and repeat 2 of the control group were 23.10% and 21.32%; the editing efficiencies of S7R protein repeat 1 and repeat 2 were 51.99% and 48.25%; the editing efficiencies of P9R protein repeat 1 and repeat 2 were 0; the editing efficiencies of Q11R protein repeat 1 and repeat 2 were 1.33% and 6.74%; the editing efficiencies of Y124R protein repeat 1 and repeat 2 were 27.46% and 30.47%; the editing efficiencies of T354R protein repeat 1 and repeat 2 were 2.53% and 4.77%; the editing efficiencies of P355R protein repeat 1 and repeat 2 were 13.40% and 10.12%.

As shown in FIG. 1, compared with the wild-type control group, mutations of P9R/Q11R/T354R/P355R site resulted in lower editing efficiency or even no editing activity of the protein; mutations of S7R/Y124R site can improve the editing efficiency of protein to some extent; this suggests that the 7th or 124th amino acid site is the key site for Cas12i3 activity.

### Example 3. Verification of editing activity of other forms of amino acid residues mutated at the 7th amino acid site of Cas protein

The results of Examples 1-2 show that the editing activity of Cas protein (as shown in SEQ ID No. 1) increased significantly after mutation at the 7th amino acid site from the N-terminus. In order to further verify the effect of the mutation of this amino acid site to other forms of amino acids on the editing activity of Cas protein, the applicant used the method of Example1 to mutate S at the 7th amino acid site to H, K, D, E, M, F, P, A, W, I, V, L, Q, C, G, N, T, or Y, respectively, and obtained the Cas protein mutated at the single amino acid site, S7A, S7N, S7D, S7C, S7Q, S7E, S7G, S7H, S7I, S7L, S7K, S7M, S7F, S7P, S7T, S7W, S7Y, or S7V Using the method of Example 1, the primer sequences used are shown in the following table:

| Amino acid mutation type (amino acid site from the N-terminus of SEQ ID No.1) | Primer name | Primer sequence |
|---|---|---|
| S7A | DT-S1056 | GTGGAGGTGGCTAGACCCTACCAGTCCCTC |
| | DT-S1055 | TAGGGTCTAGCCACCTCCACCTTCTTCTTAT |
| S7N | DT-S1057 | GTGGAGGTGAATAGACCCTACCAGTCCCTC |
| | DT-S1058 | TAGGGTCTATTCACCTCCACCTTCTTCTTAT |
| S7D | DT-S1060 | GTGGAGGTGGATAGACCCTACCAGTCCCTC |
| | DT-S1059 | TAGGGTCTATCCACCTCCACCTTCTTCTTAT |
| S7C | DT-S1062 | GTGGAGGTGTGTAGACCCTACCAGTCCCTC |
| | DT-S1061 | TAGGGTCTACACACCTCCACCTTCTTCTTAT |
| S7Q | DT-S1064 | GTGGAGGTGCAAAGACCCTACCAGTCCCTC |
| | DT-S1063 | TAGGGTCTTTGCACCTCCACCTTCTTCTTAT |
| S7E | DT-S1066 | GTGGAGGTGGAAAGACCCTACCAGTCCCTC |
| | DT-S1065 | TAGGGTCTTTCCACCTCCACCTTCTTCTTAT |
| S7G | DTT1068 | GTGGAGGTGGGAAGACCCTACCAGTCCCTC |
| | DTT1067 | TAGGGTCTTCCCACCTCCACCTTCTTCTTAT |
| S7H | DT-S1070 | GTGGAGGTGCATAGACCCTACCAGTCCCTC |
| | DT-S1069 | TAGGGTCTATGCACCTCCACCTTCTTCTTAT |
| S7I | DT-S1072 | GTGGAGGTGATAAGACCCTACCAGTCCCTC |
| | DT-S1071 | TAGGGTCTTATCACCTCCACCTTCTTCTTAT |
| S7L | DT-S1074 | GTGGAGGTGTTAAGACCCTACCAGTCCCTC |
| | DT-S0173 | TAGGGTCTTAACACCTCCACCTTCTTCTTAT |
| S7K | DTT1076 | GTGGAGGTGAAAAGACCCTACCAGTCCCTC |
| | DTT1075 | TAGGGTCTTTTCACCTCCACCTTCTTCTTAT |
| S7M | DTT1078 | GTGGAGGTGATGAGACCCTACCAGTCCCTC |
| | DTT1077 | TAGGGTCTCATCACCTCCACCTTCTTCTTAT |
| S7F | DTT1080 | GTGGAGGTGTTTAGACCCTACCAGTCCCTC |
| | DTT1079 | TAGGGTCTAAACACCTCCACCTTCTTCTTAT |
| S7P | DTT1082 | GTGGAGGTGCCTAGACCCTACCAGTCCCTC |
| | DTT1081 | TAGGGTCTAGGCACCTCCACCTTCTTCTTAT |
| S7T | DT-S1084 | GTGGAGGTGACAAGACCCTACCAGTCCCTC |
| | DT-S1083 | TAGGGTCTTGTCACCTCCACCTTCTTCTTAT |
| S7W | DT-S1086 | GTGGAGGTGTGGAGACCCTACCAGTCCCTC |
| | DT-S1085 | TAGGGTCTCCACACCTCCACCTTCTTCTTAT |
| S7Y | DT-S1088 | GTGGAGGTGTATAGACCCTACCAGTCCCTC |
| | DT-S1087 | TAGGGTCTATACACCTCCACCTTCTTCTTAT |
| S7V | DT-S1090 | GTGGAGGTGGTTAGACCCTACCAGTCCCTC |
| | DT-S1089 | TAGGGTCTAACCACCTCCACCTTCTTCTTAT |

Based on the above amino acid mutation sites, the wild-type protein (WT) of Cas12i3 and the proteins with a mutation at the single amino acid site (named after the mutation type) were obtained, respectively: S7A, S7N, S7D, S7C, S7Q, S7E, S7G, S7H, S7I, S7L, S7M, S7F, S7P, S7T, S7W, S7Y, or S7V Relative to the sequence shown in SEQ ID No. 1, the 7th amino acid from the N-terminus is mutated into A, N, D, C, Q, E, G, H, I, L, K, M, F, P, T, W, Y, or V, respectively.

For the mutant Cas protein S7A, S7N, S7D, S7C, S7Q, S7E, S7G, S7H, S7I, S7L, S7K, S7M, S7F, S7P, S7T, S7W, S7Y, or S7V, the editing activity was verified using the method of Example 2, and the results were shown in FIG. 2, and S7S in FIG. 2 is the wild-type Cas12i3 protein.

As shown in FIG. 2, the 7th amino acid of Cas12i3 was mutated to different amino acid residues, most of which significantly improved the editing activity of Cas protein. In particular, S7H, S7K, S7M, S7F, S7P, S7A, S7W, S7I, S7V, S7L, S7Q, S7C, and S7Y can significantly improve editing efficiency compared with wild type.

### Example 4. Verification of editing activity of mutant Cas protein S7R at multiple other sites

In this example, the editing activity at multiple other sites was verified for the protein S7R, which was verified in Example 2 to improve the editing efficiency of Cas protein; the editing efficiency was verified in the same way as in Example 2.

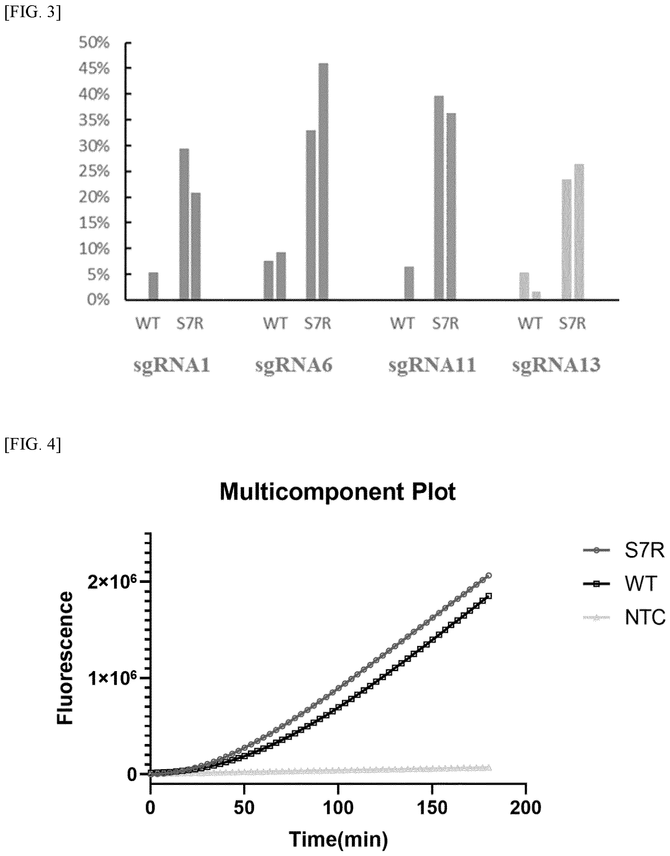

As shown in FIG. 4, the editing efficiency of S7R mutant Cas protein was significantly improved compared with wild-type Cas protein. Types of target gene editing include base deletion, base insertion, and base substitution, etc.

The targets tested include the following four targets:
Target 1: FUT8-Cas-XX-sgRNA1: TTGACAAACTGGGATACCCACCACAC;
Target 2: FUT8-Cas-XX-sgRNA6: TTGAAGCCAAGCTTCTTGGTGGTTTC;
Target 3: FUT8-Cas-XX-sgRNA11: TTGCCTCCTTTAACAAAGAAGGGTCA;
Target 4: FUT8-Cas-XX-sgRNA13: TTGTTAAAGGAGGCAAAGACAAAGTA.

### Example 5. Validation of trans activity of Cas mutant protein in vitro

In this example, trans cleavage activity of Cas protein was verified by *in vitro* detection. In this example, a gRNA that can be paired with the target nucleic acid is used to guide the Cas protein to recognize and bind to the target nucleic acid. Subsequently, Cas protein activates trans cleavage activity to any single-stranded nucleic acid, thereby cutting the single-stranded nucleic acid detector in the system; the two ends of the single-stranded nucleic acid detector are respectively provided with a fluorophore and a quenching group. If the single-stranded nucleic acid detector is cut, fluorescence will be excited; in other embodiments, the two ends of the single-stranded nucleic acid detector may also be configured as markers capable of being detected by colloidal gold.

In this experimental mode, *in vitro* trans activity was verified for the protein S7R, which was verified in Example 2 to improve the intracellular editing efficiency of Cas protein. A target N-B--g1 *TTG*CCCCCAG
CGCTTCAGCGTTC was designed for COVID19 N gene, the italic part is the PAM sequence, and the underlined area is the target region; primers were designed on two sides of target for amplification, the PCR product obtained by amplification was used as the detection template. Primer information is shown in Table 2. The gRNA sequence is AGAGAAUGUGUGCAUAGUCACACCCCCCAGCGCUUCAGCGUUC, the underlined area is the target region, other areas are DR (direct repeat sequence) regions. Verification was according to the reaction system in Table 3, 37°C incubation, and the FAM fluorescence was read per 20 s. Three repeats were set in each group, and a blank control NTC was set, that is, no target nucleic acid was added.

**Table 2. Template amplification primer information of in vitro trans activity validation**

| Name | Sequence information | Amplified fragment size |
|---|---|---|
| N-B-F | GAGGGAGCCTTGAATACACCAA | 861 bp |
| N-B-R | ATGAGTTTAGGCCTGAGTTGAG | |

**Table 3. Reaction system of Cas protein trans activity**

| **Component** | **20 µL system addition** | **Final concentration** |
|---|---|---|
| 10×T7 buffer | 2 µL | 1× |
| 10×DTT (120 mM) | 2 µL | 12 mM |
| Enzyme | 1 µL | Dilute about 100 times |
| crRNA | 0.5 µL | 10 nM |
| dsDNA | 1 uL | 10 nM |
| Reporter (10 µM) | 4 uL | 2 µM |
| H₂O | Up to 20 µL | |

The sequence of single-stranded nucleic acid detector used in this example was FAM-TTATT-BHQ1, with 5' end FAM modification and 3' end BHQ1 modification, and the fluorescence signal was read and collected by a real-time fluorescence qPCR instrument.

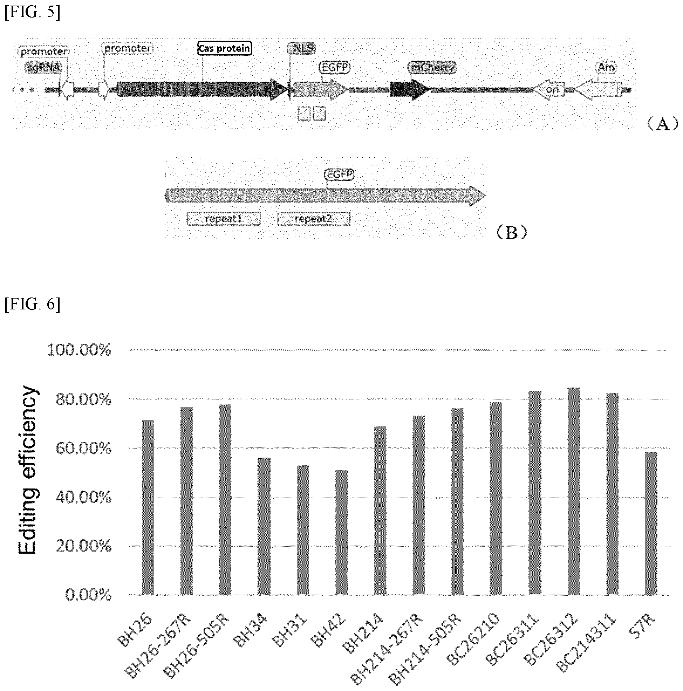

As shown in FIG. 4, compared with the control without target nucleic acid, the S7R protein can cut the single-stranded nucleic acid detection in the system and report fluorescence quickly in the presence of target nucleic acid. The above experiments reflect that the S7R protein can be used for the detection of target nucleic acids when coordinated with the single-stranded nucleic acid detector. In FIG. 5, NTC is the experimental result without the addition of target nucleic acid.

FIG. 4 also shows the comparison results of trans activity between wild-type Cas12i3 and mutant protein S7R *in vitro.* As shown in FIG. 4, S7R is mutant protein, WT is wild-type Cas12i3, and NTC is blank control. The results showed that the trans activity of mutant protein S7R was higher than that of wild-type Cas12i3 *in vitro.* It indicates that the mutation of a single site not only improved the editing activity in the cell, but also improved the detection activity *in vitro.*

### Example 6. On the basis of S7R, Cas mutant protein with improved editing activity were further obtained

Based on the mutant protein S7R obtained from the above example (amino acid sequence as shown in SEQ ID No. 3 and coding DNA sequence as shown in SEQ ID No. 4), the applicant predicted key amino acid sites that may affect its biological function through bioinformatics and further mutated and optimized combinations of amino acid sites, then the Cas mutant protein with improved editing activity was obtained.

On the basis of S7R, site-directed mutagenesis of amino acids in the Cas enzyme potentially bound to the target sequence was carried out by the bioinformatics method; variants of the Cas protein were generated by PCR-based site-directed mutagenesis, which can be performed by site-directed mutagenesis in general use in the field. The specific method is to divide the DNA sequence of the S7R protein into two parts with the mutation site as the center, design two pairs of primers to amplify the two parts of the DNA sequence, and introduce the sequence that needs to be mutated into the primers. The combination of mutants was constructed by splitting the DNA into multiple segments using PCR and Gibson clone. Fragment Amplification Kit: TransStart FastPfu DNA Polymerase (including 2.5 mM dNTPs); the specific experimental process is detailed in the manual. Gel Extraction Kit: FastPure^{®} Gel DNA Extraction Mini Kit; the specific experimental process is detailed in the manual. Kit used for vector construction: pEASY-Basic Seamless Cloning and Assembly Kit (CU201-03); the specific experimental process is detailed in the manual.

In this embodiment, mutations are made for the following sites on the basis of S7R:

| Mutant number | Amino acid mutation type (amino acid site from the N-terminus of SEQ ID No.3) |
|---|---|
| BH26 | 233R |
| BH26-267R | 233R+267R |
| BH26-505R | 233R+505R |
| BH34 | 233R+235R |
| BH31 | 168R+235R |
| BH42 | 168R+233R+235R |
| | |
| BH214 | 168R |
| BH214-267R | 168R+267R |
| BH214-505R | 168R+505R |
| BC26210 | 233R+369R+433R |
| BC26311 | 233R+267R+328R+369R |
| BC26312 | 233R+267R+369R+433R |
| BC214311 | 168R+267R+328R+369R |

Based on the above amino acid mutation sites, proteins with mutations at the following amino acid sites were obtained on the basis of S7R protein:

BH26 (the 233rd amino acid from the N-terminus of SEQ ID No. 3 is mutated to R), BH26-267R (the 233rd and 267th amino acids from the N-terminus of SEQ ID No. 3 are mutated to R), BH26-505R (the 233rd and 505th amino acids from the N-terminus of SEQ ID No. 3 are mutated to R), BH34 (the 233rd and 235th amino acids from the N-terminus of SEQ ID No. 3 are mutated to R), BH31 (the 168th and 235th amino acids from the N-terminus of SEQ ID No. 3 are mutated to R), BH42 (the 168th, 233rd, and 235th amino acids from the N-terminus of SEQ ID No. 3 are mutated to R), BH214 (the 168th amino acid from the N-terminus of SEQ ID No. 3 is mutated to R), BH214-267R (the 168th and 267th amino acids from the N-terminus of SEQ ID No. 3 are mutated to R), BH214-505R (the 168th and 505th amino acids from the N-terminus of SEQ ID No. 3 are mutated to R), BC26210 (the 233rd, 369th, and 433rd amino acids from the N-terminus of SEQ ID No. 3 are mutated to R), BC26311 (the 233rd, 267th, 328th, and 369th amino acids from the N-terminus of SEQ ID No. 3 are mutated to R), BC26312 (the 233rd, 267th, 369th, and 433rd amino acids from the N-terminus of SEQ ID No. 3 are mutated to R), and BC214311 (the 168th, 267th, 328th, and 369th amino acids from the N-terminus of SEQ ID No. 3 are mutated to R).

The different Cas proteins obtained on the basis of S7R were verified their gene editing activities in animal cells.

The vector Cas-GFFP-mCherry was constructed (the vector diagram is shown in FIG. 5), and mCherry was used for flow analysis to indicate positive transfection; the GFP gene was inserted into the target sequence and repeat sequence, resulting in gene mutation. Only the Cas protein/crRNA complex cut the target region, and the GFP gene returned to normal and gave off light after SSA (single-strand annealing) occurred in the repeat sequence, indicating that the Cas protein/crRNA complex was positive; the site targeted by gRNA was tttatctcttagggataacaggg (where ttt is the PAM sequence).

293T cells were transfected with the lipo2000 method and cultured for 48-72 h; flow analysis was performed with BD cytometry analyzer. The ratio of (GFP+mCherry+)/(GFP+mCherry+, GFP-mCherry+) of 20,000-50,000 live cells was calculated, the positive efficiency was measured, and the activity of different mutants was compared.

The results are shown in FIG. 6; compared with S7R, mutants BH26, BH26-267R, BH26-505R, BH214, BH214-267R, BH214-505R, BC26210, BC26311, BC26312, and BC214311 can significantly improve the editing activity of Cas protein. However, the editing activity of mutants BH34, BH31, and BH42 was comparable to that of S7R and even slightly decreased.

### Example 7. Editing efficiency of Cas mutant protein BC26312 in soybean

The editing efficiency of the Cas mutant protein BC26312 obtained in Example 6 (the 233rd, 267th, 369th, and 433rd amino acids from the N-terminus of SEQ ID No. 3 are mutated to R) in soybean was verified. Wild-type Cas12i3 was used as a control, which was a known Cas protein, and the amino acid sequence of wild-type Cas12i3 is shown in SEQ ID No. 1.

Gene editing in soybeans can be performed using Cas mutant protein BC26312 and wild-type Cas12i3 in a manner known in the art. In this embodiment, the methods used are as follows:

### 1. Construction of gene editing vector

gRNA for Cas protein was designed according to the coding sequences of GmFAD2-1 and GmBADH1 genes in soybean, and the designed gRNA target sequence (guide sequence) is shown in the following table.

| gRNA name | gRNA-1 | gRNA-2 |
|---|---|---|
| gRNA target sequence | cctcattgcatggccaatct | ctatggaaaccttcaagagt |
| gRNA target gene | GmFAD2-1 | GmBADH1 |

According to the direct repeat sequence of the gRNA of wild-type Cas12i3, a gRNA containing a direct repeat sequence and a guide sequence was designed. The annealing primers were designed according to the target; after the primers were annealed, the gene editing skeleton vector was connected by the Golden Gate method to obtain the gene editing vector.

### 2. Acquisition of recombinant bacteria

### 1) Escherichia coli (E. coli) transformation

The gene editing vector in step 1 was transformed into *E. coli,* and the transformed E. *coli* was subjected to bacterial liquid PCR, the amplified product with the correct PCR band size was selected for sequencing, and the *E. coli* with the correct sequencing result was the recombinant *E. coli* containing the gene editing vector.

### 2) Agrobacterium transformation

The recombinant *E. coli* containing the gene editing vector in step 1 was cultured and extracted plasmid DNA, and the plasmid DNA was added to Agrobacterium competent cells, ice bath for 5 min, liquid nitrogen for 5 min, water bath at 37 °C for 5 min, and ice bath for 5 min;
the centrifuge tube was removed, 700 µL culture solution (without antibiotics) was added, and shaking culture at 28°C for 2-4 h;
the bacterial solution was removed and coated on the medium plate containing the corresponding antibiotics, and cultured upside down in the incubator; the colonies can be seen in about 2 days, PCR was performed on the colonies according to the method in step 1), and the amplified product was sequenced; the *Agrobacterium* with the correct sequencing result was the recombinant *Agrobacterium* containing the gene editing vector.

### 3. Soybean genetic transformation

Soybean genetic transformation was carried out in the conventional way in this field, gene editing vectors containing Cas mutant protein BC26312 or wild-type Cas12i3 and the above gRNA were used to transform soybean, and E0 generation transformed seedlings were obtained.

### 4. Detection and phenotypic observation of soybean transformed strains

Edited seedling was detected and screened in the E0 generation transformed seedlings by PCR and sequencing, and planted in a climate chamber to obtain the positive seedling edited by Cas mutant protein BC26312 or wild-type Cas12i3.

### 5. Results

The gene editing vectors containing Cas mutant protein BC26312 or wild-type Cas12i3 and the above gRNA were used to transform soybean; genetically transformed positive seedlings were screened; the target gene sanger sequencing was performed on the positive seedlings; and the editing efficiency was calculated, as shown in the following table.

| | gRNA-1 | gRNA-2 |
|---|---|---|
| BC26312 editing efficiency | 65% | 60% |
| wild-type Cas12i3 editing efficiency | 1.5% | 1.0% |

The above results showed that the editing efficiency of mutant protein BC26312 at the above two targets (gRNA-1 and gRNA-2) was significantly improved compared with the wild-type Cas 12i3; the editing efficiency of wild-type Cas 12i3 can be significantly improved by mutating the 7th, 233rd, 267th, 369th, and 433rd amino acids.

### Example 8. Editing efficiency of Cas mutant protein BC26312 in CHO cells

The gene editing activity of the Cas mutant protein BC26312 obtained in Example 6 was verified in CHO cells, and wild-type Cas12i3 and spCas9 were used as controls. Targets were designed for the TTR gene target of Chinese hamster ovary cell (CHO), and 30 targets were selected to test the editing efficiency. The vector pcDNA3.3 was modified to carry EGFP fluorescent protein and PuroR resistance gene. The SV40NLS-Cas fusion protein was inserted through enzyme cutting sites XbaI and PstI; the U6 promoter and gRNA sequence were inserted through enzyme cutting site Mfe1. The CMV promoter activated the expression of fusion protein SV40NLS-Cas-XX-NLS-GFP. The protein Cas-XX-NLS was linked to the protein GFP by the linker peptide T2A. Promoter EF-1α activated the expression of the puromycin resistance gene. Seeding: seeding was conducted when the CHO cell confluency was 70-80% and the number of inoculated cells in the 12-well plate was 8×10⁴ cells/well. Transfection: transfection was conducted 24 h after seeding; 6.25 µL Hieff Trans^{™} liposome nucleic acid transfection reagent was added to 100 µL opti-MEM and mixed well; 2.5 ug plasmid was added to 100 µL opti-MEM and mixed well. The diluted Hieff Trans^{™} liposome nucleic acid transfection reagent was mixed well with the diluted plasmid and incubated at room temperature for 20 min. The incubated mixture was added to a medium with seeded cells for transfection. Screening with puromycin: puromycin was added 24 h after transfection, the final concentration was 10 µg/mL. After treatment with puromycin for 24 h, the culture medium was changed into normal medium, and the culture lasted for 24 h. 48 h after transfection, the cells were digested with trypsin-EDTA (0.05%), and the cells with GFP signal were sorted by flow cytometry (FACS).

DNA extraction, PCR amplification near the editing area, sent to hiTOM sequencing: the cells were digested by pancreatic enzyme and collected, and the genomic DNA was extracted by a cell/tissue genomic DNA extraction kit (Bioteke). The region near the target of genomic DNA was amplified. PCR products were sequenced by hiTOM. Sequencing data were analyzed, sequence types and proportions within the range of 15 nt upstream and 10 nt downstream of target position were counted, and sequences with SNV frequency greater than/equal to 1% or non-SNV mutation frequency greater than/equal to 0.06% were counted, so as to obtain the editing efficiency of different Cas proteins on target position.

The target sequence information of gRNA of the above Cas protein for TTR gene is as follows:

| gRNA target sequence information for Cas mutant protein BC26312 or wild-type Cas12i3 | gRNA target sequence information for spCas9 |
|---|---|
| TGTCTGAGGCTGGCCCTACG | TGTAGAAGGGATATACAAAG |
| ACCATCAGAGGACACTTGGA | AGTGAGTCTGGAGAGCTGCA |
| TGAACACATGCACGGCCACA | GAAGTGAGTATAAAAGCCCC |
| CCTCTGGGTAAGTTGCCAAA | GGGATTGGTGACGACAGCCG |
| CCTATAAGGTGTGAAAGTCT | GGACCTGAAGGACGAGGGAT |
| TAGAAGGGATATACAAAGTG | GCGGCAATGGTGTAGCGGCG |
| TATATCCCTTCTACAAATTC | CCAGTGGACCTGAAGGACGA |
| CACTTTGTATATCCCTTCTA | GGCTGTCGTCACCAATCCCA |
| GTGTCTATTTCCACTTTGTA | TCCAGTGGACCTGAAGGACG |
| ACCGGTGCCCTGGGTGTAGA | GGGCGGCAATGGTGTAGCGG |
| GATTCACCGGTGCCCTGGGT | GCTGCATGGGCTCACAACTG |
| TAGATGCTGTCCGAGGCAGT | GGCCGTGCATGTGTTCAGAA |
| ATGGCAGGACTGCCTCGGAC | AAAGGCTGCTGATGACACCT |
| TGAGCCCATGCAGCTCTCCA | ACACAAATACCAGTCCAGCA |
| CTCCTCAGTTGTGAGCCCAT | CCCTCGTCCTTCAGGTCCAC |
| TACAAATTCCTCCTCAGTTG | GGAGAAGTCCCTCATTCCTT |
| CTGGAAGGCACTTGGCATCT | CACATGCACGGCCACATTGA |
| CATGAGCATGCAGAGGTGAG | TAGTAAAAATGGAATACTCT |
| CACCACGGCTGTCGTCACCA | GCGGCGGGGGCCGGAGTCGT |
| AGAAAGGCTGCTGATGACAC | AAGTGCCTTCCAGTAAGATT |
| CAGTAAGATTTGGTGTCTAT | CTGCTCCTCCTCTGCCTTGC |
| GCATCTCCCCATTCCATGAG | TTGATTCTCTTTTTTTTGGA |
| TCCCTGCCAATCTGACTGCA | TTTAAAAAATCAAGTTAAAG |
| ATACTCACTTCTCCTGAGCT | GTGATGGCTGCTCCCAGCCT |
| GGAAGGGACAATAAGGGAATT | AAACACTGCTTTAGTAAAAA |
| CATGAAATCCCATCCCTCGT | AATTTTGATTCTCTTTTTTT |
| | |
| GAAGTCCAGGCAGAGACAAT | TTTTTTTGGAAGGGACAATA |
| AATATGATCTGCAGCCATTA | TGATTCTCTTTTTTTTGGAA |
| AAGTGGAATGAAAAGTGCCT | AAAATTTTACAAAGAATCAA |
| GGATCTCTCCTAGCGTTCTG | TGTGATGGCTGCTCCCAGCC |

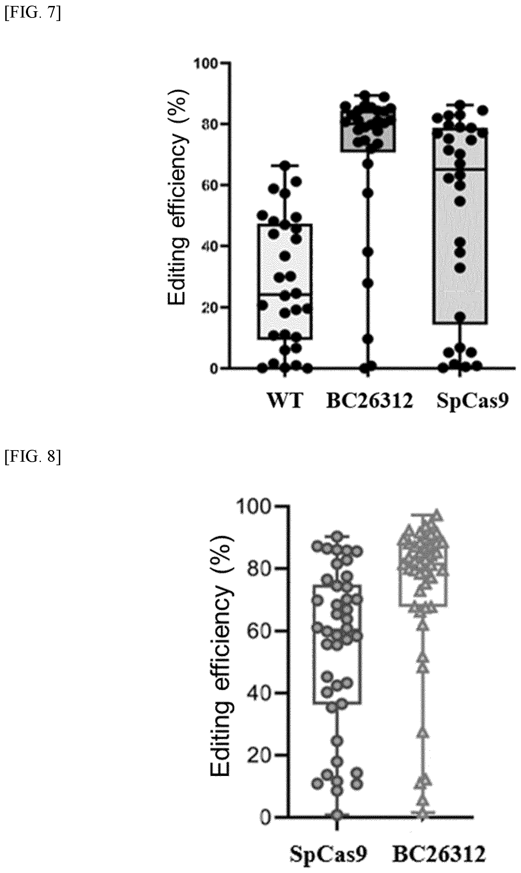

The editing efficiency of the above different Cas proteins in CHO cells was statistically analyzed. As shown in FIG. 7, the editing efficiency of Cas mutant protein BC26312 was significantly improved compared with wild-type Cas12i3 (WT in FIG. 7), and the average editing efficiency of BC26312 was better than that of SpCas9.

### Example 9. Editing efficiency of Cas mutant protein BC26312 in 293T cells

The editing efficiency of Cas mutant protein BC26312 obtained in Example 6 was verified in 293T cells in a method similar to that of Example 8, and spCas9 was used as a control. In 293T cells, 15 targets of each CCR5, PCSK9, and TTR genes were selected and constructed into the corresponding vector, and the 293T cells were transfected by lipo2000; two days after transfection, flow sorting was performed, and 50,000 cells were collected, centrifuged, and recovered; PCR amplified the target region, NGS sequencing analysis was performed, and the editing efficiency of each target was calculated.

The target sequence information of gRNA of the above Cas protein for CCR5, PCSK9, and TTR genes is as follows:

| Target gene | gRNA target sequence information for Cas mutant protein BC26312 | gRNA target sequence information for spCas9 |
|---|---|---|
| CCR5 | tgcacagggtggaacaagat | atgcacagggtggaacaaga |
| | tcaagtgtcaagtccaatct | tgacatcaattattatacat |
| | ttatacatcggagccctgcc | cctgcctccgctctactcac |
| | tacatcggagccctgccaaa | tactcactggtgttcatctt |
| | gttttgtgggcaacatgctg | ggtgttcatctttggttttg |
| | gtgggcaacatgctggtcat | tggttttgtgggcaacatgc |
| | tgggcaacatgctggtcatc | tcatcctgataaactgcaaa |
| | ccttcttactgtccccttct | tgacatctacctgctcaacc |
| | cttcttactgtccccttctg | tccttcttactgtccccttc |
| | gaaatacaatgtgtcaactc | ctcactatgctgccgcccag |
| | tataggcttcttctctggaa | gctgccgcccagtgggactt |
| | ataggcttcttctctggaat | acaatgtgtcaactcttgac |
| | taggcttcttctctggaatc | ttgacagggctctattttat |
| | ctttaaaagccaggacggtc | tattttataggcttcttctc |
| | aaagccaggacggtcacctt | tcatcctcctgacaatcgat |
| PCSK9 | ccccaggggaggacatcatt | tcatgggcaccgtcagctcc |
| | gccgctgtgtggacctcttt | ccgtcagctccaggcggtcc |
| | gggaccaactttggccgctg | tcagctccaggcggtcctgg |
| | caggtcatcacagttggggc | gctgctgctgctgctgctcc |
| | ggaaaagccagctggtccag | gctgctgctcctgggtcccg |
| | ttcggaaaagccagctggtc | tcccgcgggcgcccgtgcgc |
| | ccgaataaactccaggcctg | cgcgggcgcccgtgcgcagg |
| | cgaataaactccaggcctgg | cgcccgtgcgcaggaggacg |
| | tcgaagtcggtgaccatgac | cgtgcgcaggaggacgagga |
| | cccggtggtcactctgtatg | ggacgaggacggcgactacg |
| | ccggtggtcactctgtatgc | ggacggcgactacgaggagc |
| | atccgcccggtaccgtggag | ggtgctagccttgcgttccg |
| | cccctccacggtaccgggcg | gctagccttgcgttccgagg |
| | cccagagcatcccgtggaac | gccttgcgttccgaggagga |
| | tgtcacagagtgggacatca | gcgttccgaggaggacggcc |
| TTR | ttgacttagtcaacaaagag | cttggattcaccggtgccct |
| | tactcacttctcctgagcta | acttggattcaccggtgccc |
| | ttggcaggatggcttctcat | cagaggacacttggattcac |
| | gtgtctgaggctggccctac | tctagaactttgaccatcag |
| | agattcacgctaaatgaagt | tttgaccatcagaggacact |
| | accatcagaggacacttgga | cattgatggcaggactgcct |
| | tgaacacatgcacggccaca | tgcacggccacattgatggc |
| | gcaacttacccagaggcaaa | cacatgcacggccacattga |
| | cctataaggtgtgaaagtct | agcctttctgaacacatgca |
| | tatatcccttctacaaattc | cccagaggcaaatggctccc |
| | gtagaagggatatacaaagt | gcaacttacccagaggcaaa |
| | cactttgtatatcccttcta | ttctttggcaacttacccag |
| | ccactttgtatatcccttct | atgcagctctccagactcac |
| | gtgtctatttccactttgta | aagtgccttccagtaagatt |
| | ggtgtctatttccactttgt | acctctgcatgctcatggaa |

The editing efficiency of the above different Cas proteins in 293T cells was statistically analyzed. As shown in FIG. 8, the average editing efficiency of Cas mutant protein BC26312 was better than that of SpCas9.

Although the specific implementations of the present invention have been described in detail, those skilled in the art will understand that various modifications and changes to the details may be made in accordance with all published teachings and that these changes are within the protection scope of the present invention. The whole part of the present invention is given by the attached claims and any equivalents thereof.

### Project name: SF111PCT

Status: generated
Creation date: 2022-08-19

### SEQUENCES

### Sequence 1: "SF111-XLB_seq_1"

| Length | Molecule Type | Organism | The sequence contains both DNA & RNA fragments | Mark as an intentionally skipped sequence |
|---|---|---|---|---|
| 1045 | AA | synthetic construct | No | No |

### FEATURES

| Feature Key | Location | Qualifiers |
|---|---|---|
| REGION | 1..1045 | note = Cas12i |
| source | 1..1045 | mol_type = protein |
| | | organism = synthetic construct |

### RESIDUE

MKKVEVSRPY QSLLLPNHRK FKYLDETWNA YKSVKSLLHR FLVCAYGAVP FNKFVEVVEK 60
VDNDQLVLAF AVRLFRLVPV ESTSFAKVDK ANLAKSLANH LPVGTAIPAN VQSYFDSNFD 120
PKKYMWIDCA WEADRLAREM GLSASQFSEY ATTMLWEDWL PLNKDDVNGW GSVSGLFGEG 180
KKEDRQQKVK MLNNLLNGIK KNPPKDYTQY LKILLNAFDA KSHKEAVKNY KGDSTGRTAS 240
YLSEKSGEIT ELMLEQLMSN IQRDIGDKQK EISLPKKDVV KKYLESESGV PYDQNLWSQA 300
YRNAASSIKK TDTRNFNSTL EKFKNEVELR GLLSEGDDVE ILRSKFFSSE FHKTPDKFVI 360
KPEHIGFNNK YNVVAELYKL KAEATDFESA FATVKDEFEE KGIKHPIKNI LEYIWNNEVP 420
VEKWGRVARF NQSEEKLLRI KANPTVECNQ GMTFGNSAMV GEVLRSNYVS KKGALVSGEH 480
GGRLIGQNNM IWLEMRLLNK GKWETHHVPT HNMKFFEEVH AYNPSLADSV NVRNRLYRSE 540
DYTQLPSSIT DGLKGNPKAK ILKRQHCALN NMTANVLNPK LSFTINKKND DYTVIIVHSV 600
EVSKPRREVL VGDYLVGMDQ NQTASNTYAV MQVVKPKSTD AIPFRNMWVR FVESGSIESR 660
TLNSRGEYVD QLNHDGVDLF EIGDTEWVDS ARKFFNKLGV KHKDGTLVDL STAPRKAYAF 720
NNFYFKTMLN HLRSNEVDLT LLRNEILRVA NGRFSPMRLG SLSWTTLKAL GSFKSLVLSY 780
FDRLGAKEMV DKEAKDKSLF DLLVAINNKR SNKREERTSR IASSLMTVAQ KYKVDNAVVH 840
VVVEGNLSST DRSASKAHNR NTMDWCSRAV VKKLEDMCNL YGFNIKGVPA FYTSHQDPLV 900
HRADYDDPKP ALRCRYSSYS RADFSKWGQN ALAAVVRWAS NKKSNTCYKV GAVEFLKQHG 960
LFADKKLTVE QFLSKVKDEE ILIPRRGGRV FLTTHRLLAE STFVYLNGVK YHSCNADEVA 1020
AVNICLNDWV IPCKKKMKEE SSASG 1045

### Sequence 2: "SF111-XLB_seq_2"

| Length | Molecule Type | Organism | The sequence contains both DNA & RNA fragments | Mark as an intentionally skipped sequence |
|---|---|---|---|---|
| 3138 | DNA | synthetic construct | No | No |

### FEATURES

| Feature Key | Location | Qualifiers |
|---|---|---|
| misc_feature | 1..3138 | note = Cas12i |
| source | 1..3138 | mol_type = other DNA |
| | | organism = synthetic construct |

### RESIDUE

atgaagaagg tcgaggtgtc gcggccatac cagagcctgc tcctgccaaa ccaccggaag 60
ttcaagtacc tcgacgagac ctggaatgcg tacaagtccg ttaagagcct gctccaccgc 120
ttcctggtgt gcgcttacgg cgctgttccc ttcaacaagt tcgtggaggt tgtcgagaag 180
gttgataacg accagctcgt gctggctttc gcggtgcgcc tcttccgcct ggtccccgtg 240
gagagcacct ctttcgccaa ggttgacaag gccaatctgg cgaagtccct ggccaatcac 300
ctgcctgtgg gcacagccat tcctgccaat gttcagtcct acttcgattc aaatttcgac 360

cccaagaagt acatgtggat cgactgcgcg tgggaggctg atcgcctggc tcgggagatg 420
ggcctgagcg cgagccagtt ctctgagtac gcgactacaa tgctcctgga ggactggctg 480
cccctcaata aggatgatgt gaacggctgg gggtccgtgt cggggctctt cggcgagggc 540
aagaaggagg accggcagca gaaggtgaag atgctgaata acctgctgaa tggcatcaag 600
aagaatccgc ccaaggatta cacccagtac ctgaagatcc tcctgaacgc gttcgacgcg 660
aagtcgcaca aggaggctgt taagaactac aagggggact ctacggggcg caccgcgtct 720
tacctgtcag agaagtctgg cgagatcaca gagctgatgc tcgagcagct gatgtcaaac 780
atccagaggg atattggcga caagcagaag gagatctccc tgccgaagaa ggacgtggtc 840
aagaagtacc tcgagtcaga gtccggcgtc ccatacgatc agaacctgtg gtcccaggcc 900
taccgcaacg ctgccagctc gatcaagaag actgatacgc ggaacttcaa ctccactctc 960
gagaagttca agaatgaggt ggagctgagg ggcctgctga gcgagggcga cgacgttgag 1020
atcctgaggt ctaagttctt cagcagcgag ttccacaaga cccctgataa gttcgttatt 1080
aagccagagc atattgggtt caacaataag tacaatgtcg ttgccgagct gtacaagctc 1140
aaggctgagg cgaccgattt cgagagcgct ttcgccacag tcaaggatga gttcgaggag 1200

The residue sequence is truncated because it exceeds the specified number (1200 maximum residue to be printed)

### Sequence 3: "S7R"

| Length | Molecule Type | Organism | The sequence contains both DNA & RNA fragments | Mark as an intentionally skipped sequence |
|---|---|---|---|---|
| 1045 | AA | synthetic construct | No | No |

### FEATURES

| Feature Key | Location | Qualifiers |
|---|---|---|
| source | 1..1045 | mol_type = protein |
| | | organism = synthetic construct |

### RESIDUE

MKKVEVRRPY QSLLLPNHRK FKYLDETWNA YKSVKSLLHR FLVCAYGAVP FNKFVEVVEK 60
VDNDQLVLAF AVRLFRLVPV ESTSFAKVDK ANLAKSLANH LPVGTAIPAN VQSYFDSNFD 120
PKKYMWIDCA WEADRLAREM GLSASQFSEY ATTMLWEDWL PLNKDDVNGW GSVSGLFGEG 180
KKEDRQQKVK MLNNLLNGIK KNPPKDYTQY LKILLNAFDA KSHKEAVKNY KGDSTGRTAS 240
YLSEKSGEIT ELMLEQLMSN IQRDIGDKQK EISLPKKDVV KKYLESESGV PYDQNLWSQA 300
YRNAASSIKK TDTRNFNSTL EKFKNEVELR GLLSEGDDVE ILRSKFFSSE FHKTPDKFVI 360
KPEHIGFNNK YNVVAELYKL KAEATDFESA FATVKDEFEE KGIKHPIKNI LEYIWNNEVP 420
VEKWGRVARF NQSEEKLLRI KANPTVECNQ GMTFGNSAMV GEVLRSNYVS KKGALVSGEH 480
GGRLIGQNNM IWLEMRLLNK GKWETHHVPT HNMKFFEEVH AYNPSLADSV NVRNRLYRSE 540
DYTQLPSSIT DGLKGNPKAK LLKRQHCALN NMTANVLNPK LSFTINKKND DYTVIIVHSV 600
EVSKPRREVL VGDYLVGMDQ NQTASNTYAV MQVVKPKSTD AIPFRNMWVR FVESGSIESR 660
TLNSRGEYVD QLNHDGVDLF EIGDTEWVDS ARKFFNKLGV KHKDGTLVDL STAPRKAYAF 720
NNFYFKTMLN HLRSNEVDLT LLRNEILRVA NGRFSPMRLG SLSWTTLKAL GSFKSLVLSY 780
FDRLGAKEMV DKEAKDKSLF DLLVAINNKR SNKREERTSR IASSLMTVAQ KYKVDNAVVH 840
VVVEGNLSST DRSASKAHNR NTMDWCSRAV VKKLEDMCNL YGFNIKGVPA FYTSHQDPLV 900
HRADYDDPKP ALRCRYSSYS RADFSKWGQN ALAAVVRWAS NKKSNTCYKV GAVEFLKQHG 960
LFADKKLTVE QFLSKVKDEE ILIPRRGGRV FLTTHRLLAE STFVYLNGVK YHSCNADEVA 1020
AVNICLNDWV IPCKKKMKEE SSASG 1045

### Sequence 4: "S7R"

| Length | Molecule Type | Organism | The sequence contains both DNA & RNA fragments | Mark as an intentionally skipped sequence |
|---|---|---|---|---|
| 3135 | DNA | synthetic construct | No | No |

### FEATURES

| Feature Key | Location | Qualifiers |
|---|---|---|
| source | 1..3135 | mol_type = other DNA |
| | | organism = synthetic construct |

### RESIDUE

atgaagaagg tcgaggtgag gcggccatac cagagcctgc tcctgccaaa ccaccggaag 60
ttcaagtacc tcgacgagac gtggaatgcg tacaagtccg ttaagagcct gctccaccgc 120
ttcctggtgt gcgcttacgg cgctgttccc ttcaacaagt tcgtggaggt tgtcgagaag 180
gttgataacg accagctcgt gctggctttc gcggtgcgcc tcttccgcct ggtccccgtg 240
gagagcacct ctttcgccaa ggttgacaag gccaatctgg cgaagtccct ggccaatcac 300
ctgcctgtgg gcacagccat tcctgccaat gttcagtcct acttcgattc aaatttcgac 360
cccaagaagt acatgtggat cgactgcgcg tgggaggctg atcgcctggc tcgggagatg 420
ggcctgagcg cgagccagtt ctctgagtac gcgactacaa tgctctggga ggactggctg 480
cccctcaata aggatgatgt gaacggctgg gggtccgtgt cggggctctt cggcgagggc 540
aagaaggagg accggcagca gaaggtgaag atgctgaata acctgctgaa tggcatcaag 600
aagaatccgc ccaaggatta cacccagtac ctgaagatcc tcctgaacgc gttcgacgcg 660

aagtcgcaca aggaggctgt taagaactac aagggggact ctacggggcg caccgcgtct 720
tacctgtcag agaagtctgg cgagatcaca gagctgatgc tcgagcagct gatgtcaaac 780
atccagaggg atatcggcga caagcagaag gagatctccc tgccgaagaa ggacgtggtc 840
aagaagtacc tggagtcaga gtccggcgtc ccatacgatc agaacctgtg gtcccaggcc 900
taccgcaacg ctgccagctc gatcaagaaa actgatacgc ggaacttcaa ctccactctg 960
gagaagttca agaatgaggt ggagctgagg ggcctcctga gcgagggcga cgacgttgag 1020
atcctgaggt ctaagttctt cagcagcgag ttccacaaga cccctgataa gttcgttatt 1080
aagccagagc atattgggtt caacaataag tacaatgtcg ttgccgagct gtacaagctc 1140
aaggctgaag cgaccgattt cgagagtgct ttcgccacag tcaaggatga gttcgaggag 1200

The residue sequence is truncated because it exceeds the specified number (1200 maximum residue to be printed)

## Claims

1. A clustered regularly interspaced short palindromic repeats (CRISPR)-associated protein (Cas) mutant protein, wherein compared to an amino acid sequence of a parent Cas protein, the mutant protein has mutations at any one or more of the following amino acid sites corresponding to the amino acid sequence shown in SEQ ID No. 1: 7th and 124th sites.

2. The Cas mutant protein according to claim 1, wherein a 7th amino acid is mutated to an amino acid other than S, for example, R, H, K, M, F, P, A, W, I, V, L, Q, C, or Y; a 124th amino acid is mutated to an amino acid other than Y, for example, R, H, K, M, F, P, A, W, I, V, L, Q, or C.

3. The Cas mutant protein according to claim 1 or 2, wherein compared to the amino acid sequence of the parent Cas protein, the mutant protein has a mutation at the 7th amino acid site corresponding to the amino acid sequence shown in SEQ ID No. 1.

4. The Cas mutant protein according to claim 3, wherein compared to the amino acid sequence of the parent Cas protein, the mutant protein further has mutations at any one or more of the following amino acid sites corresponding to the amino acid sequence shown in SEQ ID No. 1: 233rd, 267th, 369th, 433rd, 168th, 328th, and 505th sites.

5. The Cas mutant protein according to claim 4, wherein compared to the amino acid sequence of the parent Cas protein, the mutant protein further has mutations at the following amino acid sites corresponding to the amino acid sequence shown in SEQ ID No. 1:
a 233rd amino acid is mutated;
or, the 233rd amino acid and a 267th amino acid are mutated simultaneously;
or, the 233rd amino acid, a 369th amino acid, and a 433rd amino acid are mutated simultaneously;
or, the 233rd amino acid, the 267th amino acid, the 369th amino acid, and the 433rd amino acid are mutated simultaneously;
or, the 233rd amino acid and a 505th amino acid are mutated simultaneously;
or, the 233rd amino acid, the 267th amino acid, a 328th amino acid, and the 369th amino acid are mutated simultaneously;
or, a 168th amino acid is mutated;
or, the 168th amino acid and the 267th amino acid are mutated simultaneously;
or, the 168th amino acid and the 505th amino acid are mutated simultaneously;
or, the 168th amino acid, the 267th amino acid, the 328th amino acid, and the 369th amino acid are mutated simultaneously.

6. The Cas mutant protein according to any one of claims 1-5, wherein the parent Cas protein is a Cas protein of a Cas12i family.

7. The Cas mutant protein according to any one of claims 1-3, wherein the Cas mutant protein is selected from any one of the following I-III groups:
I, a Cas mutant protein by mutating the amino acid sequence shown in SEQ ID No. 1 at any one or more of the following amino acid sites: the 7th and 124th sites;
II, a Cas mutant protein with a mutation site described in I and has a sequence identity of at least 80% compared to the Cas mutant protein described in I; and
III, a sequence with the mutation site described in I compared to the Cas mutant protein described in I; and the sequence has a substitution, a deletion, or an addition of one or more amino acids compared with the Cas mutant protein described in I;
wherein the one or more amino acids comprise the substitution, the deletion, or the addition of one, two, three, four, five, six, seven, eight, nine, or ten amino acids.

8. The Cas mutant protein according to any one of claims 4-5, wherein the Cas mutant protein is selected from any one of the following i-iii groups:
i, a Cas mutant protein obtained from a mutation in the amino acid sequence shown in SEQ ID No. 1 at the 7th amino acid site and a mutation at any one or more of the following amino acid sites in the amino acid sequence shown in SEQ ID No. 1: 233rd, 267th, 369th, 433rd, 168th, 328th, and 505th sites;
ii, a Cas mutant protein with a mutation site described in i; and the Cas mutant protein having a sequence identity of at least 80% compared to the Cas mutant protein described in i; and
iii, a sequence with the mutation site described in i; and the sequence having a substitution, a deletion, or an addition of one or more amino acids compared with the Cas mutant protein described in i; wherein the one or more amino acids comprise the substitution, the deletion, or the addition of one, two, three, four, five, six, seven, eight, nine, or ten amino acids.

9. The Cas mutant protein according to any one of claims 4-5, wherein the Cas mutant protein is selected from any one of the following a-c groups:
a, a Cas mutant protein by mutating the amino acid sequence shown in SEQ ID No. 3 at any one or more of the following amino acid sites: 233th, 267th, 369th, 433rd, 168th, 328th, and 505th sites; and any one or more of amino acid sites in the Cas mutant protein corresponding to 7th, 233rd, 267th, 369th, 433rd, 168th, 328th, or 505th site in SEQ ID No. 3 being R;
b, a Cas mutant protein with a mutation site described in a; and the Cas mutant protein having a sequence identity of at least 80% compared to the Cas mutant protein described in a; and
c, a sequence with the mutation site described in a; and the sequence having a substitution, a deletion, or an addition of one or more amino acids compared with the Cas mutant protein described in a; wherein the one or more amino acids comprise the substitution, the deletion, or the addition of one, two, three, four, five, six, seven, eight, nine, or ten amino acids.

10. A fusion protein, comprising the Cas mutant protein according to any one of claims 1-9 and other modification parts; wherein for example, the modification parts are selected from another protein or polypeptide, a detectable marker, or any combination thereof.

11. An isolated polynucleotide, wherein the polynucleotide is a polynucleotide sequence encoding the Cas mutant protein according to any one of claims 1-9 or a polynucleotide sequence encoding the fusion protein according to claim 10.

12. A vector, comprising the polynucleotide according to claim 11 and a regulatory element operably linked to the polynucleotide.

13. A CRISPR-Cas system, comprising the Cas mutant protein according to any one of claims 1-9 and at least one gRNA;
wherein the gRNA is capable of binding to the Cas mutant protein according to any one of claims 1-9.

14. A composition, comprising:
(i) a protein component selected from the Cas mutant protein according to any one of claims 1-9 or the fusion protein according to claim 10; and
(ii) a nucleic acid component, wherein the nucleic acid component is a gRNA capable of binding to the Cas mutant protein according to any one of claims 1-9;
wherein the protein component combines with the nucleic acid component to form a complex.

15. An activated CRISPR complex, comprising:
(i) a protein component selected from the Cas mutant protein according to any one of claims 1-9 or the fusion protein according to claim 10;
(ii) a nucleic acid component, wherein the nucleic acid component is a gRNA comprising a direct repeat sequence capable of binding to the Cas mutant protein according to any one of claims 1-9 and a guide sequence capable of targeting a target sequence; and
(iii) the target sequence bound to the gRNA in (ii).

16. An engineered host cell, comprising the Cas mutant protein according to any one of claims 1-9, or the fusion protein according to claim 10, or the polynucleotide according to claim 11, or the vector according to claim 12, or the CRISPR-Cas system according to claim 13, or the composition according to claim 14, or the activated CRISPR complex according to claim 15.

17. An application of the Cas mutant protein according to any one of claims 1-9, or the fusion protein according to claim 10, or the polynucleotide according to claim 11, or the vector according to claim 12, or the CRISPR-Cas system according to claim 13, or the composition according to claim 14, or the activated CRISPR complex according to claim 15, or the host cell according to claim 16 in a gene editing, a gene targeting, or a gene cleavage; or in a preparation of a reagent or a kit for the gene editing, the gene targeting, or the gene cleavage.

18. An application of the Cas mutant protein according to any one of claims 1-9, or the fusion protein according to claim 10, or the polynucleotide according to claim 11, or the vector according to claim 12, or the CRISPR-Cas system according to claim 13, or the composition according to claim 14, or the activated CRISPR complex according to claim 15 in any one or more of the following:
targeting and/or editing a target nucleic acid; a cleavage of a double-stranded DNA, a single-stranded DNA, or a single-stranded RNA; a non-specific cleavage and/or degradation of a collateral nucleic acid; a non-specific cleavage of a single-stranded nucleic acid; a nucleic acid detection; a specific editing of a double-stranded nucleic acid; a base editing of the double-stranded nucleic acid; and a base editing of the single-stranded nucleic acid.

19. A kit for a gene editing, a gene targeting, or a gene cleavage, comprising the Cas mutant protein according to any one of claims 1-9, or the fusion protein according to claim 10, or the polynucleotide according to claim 11, or the vector according to claim 12, or the CRISPR-Cas system according to claim 13, or the composition according to claim 14, or the activated CRISPR complex according to claim 15.

20. A use of the Cas mutant protein according to any one of claims 1-9, or the fusion protein according to claim 10, or the polynucleotide according to claim 11, or the vector according to claim 12, or the CRISPR-Cas system according to claim 13, or the composition according to claim 14, or the activated CRISPR complex according to claim 15 in a preparation of a preparation or a kit, wherein the preparation or the kit is used for:
(i) a gene or genome editing;
(ii) a target nucleic acid detection and/or diagnosis;
(iii) editing a target sequence in a target locus to modify an organism;
(iv) a treatment of a disease;
(v) targeting a target gene; and
(vi) cutting the target gene.
